(19) Europäisches Patentamt
European Patent Office
Office européen des brevets

(11) Veröffentlichungsnummer: **0 280 915 A2**

# (12) EUROPÄISCHE PATENTANMELDUNG

(21) Anmeldenummer: **88101673.7**

(22) Anmeldetag: **05.02.88**

(51) Int. Cl.⁴: **A61K 31/57**, //(A61K31/57, 31:47),(A61K31/57,31:535), (A61K31/57,31:495)

(30) Priorität: **17.02.87 DE 3704907**

(43) Veröffentlichungstag der Anmeldung: **07.09.88 Patentblatt 88/36**

(84) Benannte Vertragsstaaten: **AT BE CH DE ES FR GB GR IT LI NL SE**

(71) Anmelder: **BAYER AG**
**Konzernverwaltung RP Patentabteilung**
**D-5090 Leverkusen 1 Bayerwerk(DE)**

(72) Erfinder: **Grohe, Klaus, Dr.**
**Am Wasserturm 10**
**D-5068 Odenthal(DE)**

(54) **Topisch anwendbare Zubereitungen von Gyrase-Inhibitoren in Kombination mit Kortikosteroiden.**

(57) Die Erfindung betrifft topisch anwendbare Zubereitungen, die als Wirkstoffe zur Gruppe der Gyrase-Inhibitoren zählende antibakteriell wirksame Verbindungen der Formel

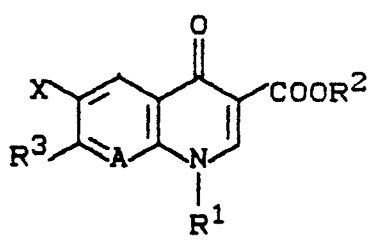

(I)

in welcher $R^1$, $R^2$, $R^3$, X und A die in der Beschreibung angegebene Bedeutung haben, und ein Kortikosteroid oder mehrere Kortikosteroide enthalten.

EP 0 280 915 A2

## Topisch anwendbare Zubereitungen von Gyrase-Inhibitoren in Kombination mit Kortikosteroiden

Die Erfindung betrifft topisch anwendbare Zubereitungen, die als Wirkstoffe zur Gruppe der Gyrase-Inhibitoren zählende antibakteriell wirksame Verbindungen der allgemeinen Formel

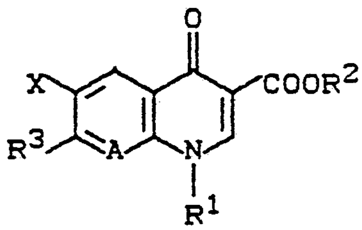

(I)

in welcher

$R^1$ für Methyl, Ethyl, Propyl, Isopropyl, Cyclopropyl, Vinyl, 2-Hydroxyethyl, 2-Fluorethyl, Methoxy, Amino, Methylamino, Dimethylamino, Ethylamino, Phenyl, 4-Fluorphenyl, 2,4-Difluorphenyl,

$R^2$ für Wasserstoff, Alkyl mit 1 bis 4 Kohlenstoffatomen, (5-Methyl-2-oxo-1,3-dioxol-4-yl)-methyl,

$R^3$ für Methyl oder eine cyclische Aminogruppe wie

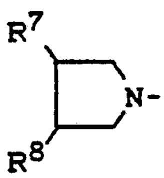 , 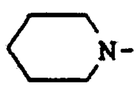 , 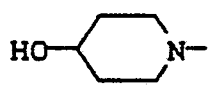 , 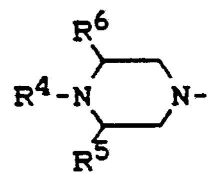 ,

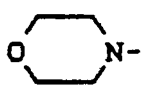 , 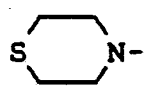 , 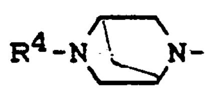 , 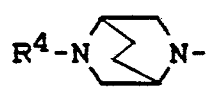 ,

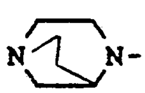 , 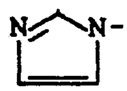 , 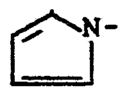

stehen, worin

$R^4$ für Wasserstoff, Alkyl mit 1 bis 4 Kohlenstoffatomen, 2-Hydroxyethyl, Allyl, Propargyl, 2-Oxopropyl, 3-Oxobutyl, Phenacyl, Formyl, $CFCl_2$-S-, $CFCl_2$-$SO_2$-, $CH_3O$-CO-S-, Benzyl, 4-Aminobenzyl,

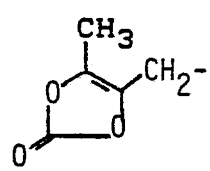

$R^5$ für Wasserstoff, Methyl,

$R^6$ für Wasserstoff, Alkyl mit 1 bis 4 Kohlenstoffatomen, Phenyl, Benzyloxymethyl,

$R^7$ für Wasserstoff, Amino, Methylamino, Ethylamino, Aminomethyl, Methylaminomethyl, Ethylaminomethyl, Dimethylaminomethyl, Hydroxy, Hydroxymethyl,

$R^8$ für Wasserstoff, Methyl, Ethyl oder Chlor stehen,

X für Wasserstoff, Fluor, Chlor oder Nitro und

A für N oder C-$R^9$ stehen, worin

$R^9$ für Wasserstoff, Halogen wie Fluor oder Chlor, Methyl oder Nitro steht,

oder

A auch gemeinsam mit $R^1$ eine Brücke der Struktur $-O-CH_2-\underset{|}{C}H-CH_3$, $-S-CH_2-\underset{|}{C}H-CH_3$ oder $-CH_2-CH_2-\underset{|}{C}H-CH_3$ bilden kann,

und ein Kortikosteroid oder mehrere Kortikosteroide enthalten.

Die Gyrase-Inhibitoren können in den topisch anwendbaren Zubereitungen als solche oder als Salz mit einer Säure oder Base angewendet werden. Auch die Verwendung als Prodrug, beispielsweise von Estern, ist möglich.

Die Gyrase-Inhibitoren werden in Verbindung mit Kortikosteroiden in Form der topisch anwendbaren Zubereitungen zur Behandlung oder Prophylaxe von Infektionen, Erkrankungen und Verletzungen der Haut einschließlich Verbrennungen topisch appliziert. Auch eine Behandlung oder Prophylaxe tiefer gelegener oder systemischer Infektionen ist durch eine topische Applikation von Gyrase-Inhibitoren möglich.

Die erfindungsgemäßen topisch anwendbaren Zubereitungen enthalten 0,05 bis 30, bevorzugt 0,05 bis 20 Gew.-% Wirkstoff der Formel (I) und Kortikosteroide.

Besonders bevorzugt enthalten die erfindungsgemäßen topisch anwendbaren Zubereitungen 0,1 bis 5 Gew.-% Wirkstoff der Formel (I) und Kortikosteroide.

Insbesondere sind Ciprofloxacin, Norfloxacin, Pefloxacin, Amifloxacin, Pirfloxacin, Ofloxacin und/oder Enoxacin in den genannten Zubereitungen enthalten.

Die Kortikosteroid-Wirkstoffe, die in den erfindungsgemäßen Zuvereitungen zur Anwendung kommen sind bekannt. Solche Wirkstoffe werden detailliert, beispielsweise in Miller, Zunro, Dengs 19, 119-134 (1980) und Wolfe, Bayer's Medicinal Chemistry 3 4A Ed., John Wiley and Bus, New York, N.Y. Seiten 1273-1316, 917-1309 (1981) beschrieben.

Weitere Kortikosteroid-Wirkstoffe, die als Bestandteile der erfindungsgemäßen Zubereitungen infrage kommen, sind beschrieben in: EP 0 036 138, EP 0 129 283, EP 0 098 566, EP 0 173 478, EP 0 136 586, EP 0 098 568, EP 0 095 894, EP 0 078 235, EP 0 023 713, DE 3 227 312, DE 3 243 482, DE 3 401 680, DE 3 400 188, US 4 257 969 und US 4 343 798.

Bevorzugte Kortikosteroide sind Hydroxytriamcinolon, α-Methyl-dexamethason, β-Methyl-betamethason, Beclomethason-α-propionat, Betamethasonbenzoat, Betamethasondipropionat, Betamethasonvalerat, Clobetasolvalerat, Desonid, Desoxymethason, Dexamethason, Diflorasondiacetat, Diflucortolonvalerat, Flurandrenolon, Fluclorolonacetonid, Flumethasonpivalat, Fluocinolonacetonid, Fluocinonid, Fluocortinebutylester, Fluocortolon, Fluprednidenacetat, Halcinonid, Hydrocortisonacetat, Hydrocortisonbutyrat, Methylprednisolon, Triamcinolonacetonid, Cortison, Cortodoxon, Flucetonid, Fludrocortison, Diflorasondiacetat , Fluradrenolonacetonid, Medryson,Amcinafal , Amcinafid, Betamethason sowie dessen Ester, Chloroprednison, Clocortolon, Clescinolon, Dichlorison, Difluprednat, Flucloronid, Flunisolid, Fluoromethalon, Fluperolon, Fluprednisolon, Hydrocortison, Meprednison, Paramethason, Prednisolon, Prednison sowie Beclomethasondipropionat.

Auch Mischungen des sogenannten Kortikosteroide sind als Bestandteile der erfindungsgemäßen Zubereitungen möglich.

Beispiele für die erfindungsgemäß einzusetzenden Kortikosteroide sind in bevorzugten Gew.-% insbesondere:

A Beclomethasondipropionat 0,5 %
B Clobetasolpropionat 0,05 %
C Diflucortolonvalerat 0,3 %
D Fluocinolonacetonid 0,2 %
E Beclomethasondipropionat 0,025 %
F Betamethasonbenzoat 0,025 %
G Betamethasondipropionat 0,05 %
H Betamethasonvalerat 0,1 %
I Desonid 0,05 %
J Desoxymethason 0,25 %
K Difluorasondiacetat 0,05 %
L Diflucortolonvalerat 0,1 %
M Fluclorolonacetonid 0,025 %
N Fluocinolonacetonid 0,025 %
O Fluocinonid 0,05 %
P Fluocortolon 0,5 %
Q Flupredniden (Fluprednyliden)acetat 0,1 %
R Flurandrenolon 0,05 %

S Halcinonid 0,1 %
T Hydrocortisonbutyrat 0,1 %
X Triamcinolonacetonid 0,1 %
Y Clobetasonbutyrat 0,05 %
Z Flumethasonpivalat 0,02 %
A[1] Fluocinolonacetonid 0,01 %
B[1] Fluocortinbutylester 0,75 %
C[1] Fluocortolon 0,2 %
D[1] Flurandrenalon 0,0125 % - 0,025 %
E[1] Hydrocortison (Harnstoff) 1 %
F[1] Dexamethason 0,01 %
G[1] Hydrocortison (Alcohol oder Acetat) 0,1 % - 1 %
H[1] Methylprednisolon 0,25 %

Besonders bevorzugt sind Kortikosteroide aus der Gruppe bestehend aus Triamcinolonacetonid , Hydrocortisonacetat, Betamethasonvalerat, Fluocinolonacetonid, Flupameson und Mischungen dieser Verbindungen.

Andere bevorzugte Wirkstoffe sind Desoxycorticosteron, Fludrocortison, Hydrocortison, Betamethason, Cortison, Dexamethason, Prednisolon, Prednison, Methylprednisolon, Paramethason, Triamcinolon und Mischungen dieser Verbindungen.

Die erfindungsgemäßen Zubereitungen enthalten die Kortikoidsteroide in Mengen von 0,01 bis 10 Gew.-%, bevorzugt 0,02 bis 5 Gew.-% und besonders bevorzugt 0,05 bis 5 Gew.-%.

## A. Allgemeiner Teil

Zu den topischen Zubereitungen der Erfindung gehören Lösungen, Sprays, Lotionen, Gele, Salben, Cremes, Pulver, Pudersprays, Pasten, Suspensionen, Emulsionen, Schäume und Stifte, die den Wirkstoff der Formel I, gegebenenfalls auch mehrere Wirkstoffe, enthalten.

Die topische Applikation der vorliegenden Verbindungen der Formel I erfolgt auch in Form von Pflastern, Sprühpflastern, Okklusivverbänden, Umschlägen und gesteuerten Abgabesystemen. In diesen Zubereitungen können die Wirkstoffe in gelöster oder suspendierter Form enthalten sein.

Salben enthalten als Grundlage Kohlenwasserstoffgele, Lipogele, Absorptionsgrundlagen, W/O-Salbengrundlagen, Mischemulsionen oder Polyethylenglykole.

Cremes enthalten O/W-Grundlagen.

Pasten enthalten neben einer Salben-oder Cremegrundlage hohe Anteile pulverförmiger Bestandteile wie Zinkoxid, Talk, Stärke oder Titandioxid.

Gele enthalten Lösungsmittel wie Wasser, Ethanol, Isopropanol oder Propylenglykol und werden unter Verwendung von Gelbildnern wie Celluloseethern, Alginaten, Polyacrylaten, Bentonit, Gelatine, Tragant, Polyvinylpyrrolidon der Polyvinylalkohol hergestellt. Auch die Verwendung lipophiler Gelgrundlagen oder von Mikroemulsionen ist möglich.

Puder enthalten pulverförmige Zusatzstoffe wie Stärke, Stearate, Siliciumdioxid, Ton, Magnesiumcarbonat, Talk, Cellulose, Zinkoxid und insbesondere Lactose.

Allen Zubereitungen können Stabilisatoren, Antioxidantien, Konservierungsmittel, Feuchthaltemittel, Rückfetter, Lösungsmittel oder Hilfsstoffe zur Verbesserung von Penetration und Wirksamkeit zugesetzt werden.

Beispiele von Penetrationsverbesserern sind Propylenglykol, Polyethylenglykol, Dimethylsulfoxid, Decylmethylsulfoxid, Azone, N-Methyl-pyrrolidon, Diethyltoluamid, Ethanol, Isopropylmyristat, Isopropylpalmitat, Ölsäure und seine Ester, mittelkettige Triglyceride, Dimethylisosorbit, 2-Octyldodecanol, verzweigtkettige Fettsäureester, Benzylalkohol, Harnstoff, Salicylate und Tenside.

## B. Haftende topische Zubereitungen

Soweit es sich um die Behandlung von Tieren handelt, hat es sich als vorteilhaft erwiesen, haftende topisch anwendbare Zubereitungen zu verwenden und die Erfindung wie im folgenden beispielhaft erläutert auszuführen.

Erfindungsgemäße topische Zubereitungen, die sowohl auf nasse wie trockene Tiere aufgetragen werden können, sind beispielsweise dadurch gekennzeichnet, daß sie

a) 0,1 - 20 %, vorzugsweise 0,1 - 5 %, eines Wirkstoffs der Formel I,

$a_1$) 0,01 bis 10 % eines Kortikosteroids,

b) 1 - 40 %, vorzugsweise 1 - 20 %, eines wasserlöslichen Gel-oder Lack-bildenden Polymers,

c) 40 -98 %, vorzugsweise 60 - 90 %, eines organischen, wassermischbaren Lösungsmittel, das - schneller verdunstet als Wasser und in dem sich das Polymer nicht löst,

c) 0,1 - 10 %, verschiedener Additiva, z.B. Weichmacher, Suspendierhilfsmittel, Antioxydantien, Spreitmittel, Farbstoffe etc. enthalten.

Zur Herstellung der Zubereitungen werden an sich bekannte Polymere oder deren Salze in einem Lösungsmittel suspendiert, in dem sie nicht löslich sind. Die Polymeren verquellen dagegen in Wasser zu einem Gel. Der Wirkstoff wird in dem Lösungsmittel entweder suspendiert oder gelöst. Das Lösungsmittel muß mit Wasser mischbar sein und schneller als Wasser verdunsten können. Der Suspension können die üblichen Formulierhilfsmittel zugesetzt werden, um eine leicht aufschüttelbare oder homogene Suspension sicherzustellen. Ein Weichmacherzusatz kann ebenfalls erwünscht sein, um den sich bildenden Film später elastisch zu halten.

Wird eine solche Suspension auf ein nasses Tier gegossen oder gesprüht, so verquillt das Polymer mit dem Verdunsten des Lösungsmittels zu einem Gel, das zu einer Lack-oder Filmschicht austrocknet und dabei den Wirkstoff inkorporiert. Diese Schicht bleibt lange Zeit auf dem Haarkleid bzw. der Haut haften und wird durch Regengüsse oder ein Dipbad nur langsam - nach und nach - abgewaschen.

Die Suspension wird im etwa gleichen Verhältnis mit Wasser verdünnt. Sie ist dann noch niedrigviskos, das Polymer noch nicht verquollen, so daß sie sich mit den üblichen Geräten mühelos applizieren läßt. Auch hier bildet sich nach dem Verdunsten des Lösungsmittels ein Gel und später ein Film aus, wie bereits oben beschrieben.

Als Gel-und Filmbildner kommen alle makromolekularen Verbindungen in Frage, die sich in dem wassermischbaren, organischen Lösungmittel nicht lösen und nach Mischen mit Wasser zu einem Gel verquellen, das nach dem Trocknen eine Art Film gibt.

Folgt man einer Einteilung der makromolekularen Hilfsstoffe, wie z.B. von Keipert et al. in Die Pharmazie 28, 145-183 (1973) beschrieben, so kommen vor allem ionische Makromoleküle in deren Salzform zur Anwendung. Dies sind unter anderem Natriumcarboxymethylcellulose, Polyacrylsäure, Polymethacrylsäure und deren Salze, Natriumamylopektinsemiglykolat, Alginsäure und Propylenglykol-Alginat als Natriumsalz, arabisches Gummi, Xanthan-Gummi und Guar-Gummi.

Amphotere Makromoleküle wie Protein-Derivate, z.B. Gelatine sind ebenso geeignet wie nichtionische Polymere z.B. Methylcellulose, andere Cellulose-Derivate und lösliche Stärken, die obige Anforderungen erfüllen.

Als Lösungsmittel sind alle mit Wasser mischbaren Flüssigkeiten geeignet, die das Makromolekül nicht lösen und schneller verdunsten als Wasser.

In Betracht kommen z.B. Alkanole wie Ethanol und Isopropylalkohol; Ketone wie Aceton, Methylethylketon, Glykolether wie Ethylenglykolmonomethylether oder -ethylether.

Es können bei der Herstellung der erfindungsgemäßen Zubereitungen der oben beschriebenen Art ein oder mehrere Lösungsmittel eingesetzt werden.

Als weitere Hilfsmittel für solche Zubereitungen sind geeignet:

a) Substanzen, die die Suspension stabilisieren können, z.B. kolloidale Kieselsäure, Montmorillonite u.a.

b) Tenside (beinhaltet Emulgatoren und Netzmittel), z.B.

1. anionaktive, wie Na-Laurylsulfat, Fettalkoholethersulfate, Mono/Dialkylpolyglykoletherorthophosphorsäureester-Monoethanolaminsalz;

2. kationaktive, wie Cetyltrimethylammoniumchlorid;

3. ampholytische, wie Di-Na-N-lauryl-$\beta$-iminodipropionat oder Lecethin;

4. nicht ionogene, z.B. polyoxyethyliertes Rizinusöl, polyoxyethyliertes Sorbitan-Monooleat, Sorbitan-Monostearat, Cetylalkohol, Glycerinmonostearat, Polyoxyethylenstearat, Alkylphenolpolyglykolether.

c) Stabilisatoren zur Verhinderung des bei einigen Wirkstoffen eintretenden chemischen Abbaues wie Antioxydatien, z.B. Tocopherole, Butylhydroxyanisol.

d) Weichmacher für die Elastizität der Filmbildner, z.B. Glycerin, Propylenglykol.

Für die Anwendung der erfindungsgemäßen topischen Zubereitungen zur Bekämpfung bakterieller Erkrankungen des Menschen kommen bevorzugt als Anwendungsformen alle in der Dermatologie und Kosmetik üblichen Formulierungen in Betracht. Besonders zu erwähnen sind solche, die nach Auftragen auf die Haut bei Kontakt mit Wasser nicht gleich abgewaschen werden, also solche, die filmbildende oder

wasserabweisende Zusatzstoffe wie die bereits oben erwähnten Zubereitungen enthalten. Formulierungen dieser Art sind z.B. Lösungen, Sprays, Lotionen, Salben (hier sowohl Emulsionssalben als auch Suspensionssalben und Stifte analog Insekten-Repellent-Stiften).

C. Flüssige Zubereitungen mit Spreitmitteln

Den erfindungsgemäßen Zubereitungen können, soweit sie in flüssiger Form vorliegen, zur besseren Verteilung auf Oberflächen, insbesondere auf der Haut, auch spreitende Öle zugesetzt werden.

Unter spreitenden Ölen werden solche öligen Flüssigkeiten verstanden, die sich auf der Haut besonders gut verteilen. Sie sind als solche in der Kosmetik bekannt. Nach einem Vorschlag von R. Reymer, Pharm. Ind. 32, 577 (1970) können sie z.B. durch ihre Oberflächenspannung gegen Luft charakterisiert werden, die danach weniger als 30 dyn/cm betragen sollte.

Als Spreitmittel kommen besonders die folgenden Substanzen in Betracht:

Silikonöle verschiedener Viskosität

Fettsäureester wie Ethylstearat, Di-n-butyl-adipat , Laurinsäurehexylester, Dipropylen-glykolpelargonat, Ester einer verzweigten Fettsäure mittlerer Kettenlänge mit gesättigten Fettalkoholen $C_{16}$-$C_{18}$, Isopropylmyristat, Isopropylpalmitat, Capryl/Caprinsäureester von gesättigten Fettalkoholen der Kettenlänge $C_{12}$-$C_{18}$, Isopropylstearat, Ölsäureoleylester, Ölsäuredecylester, Ethyloleat, Milchsäureethylester, wachsartige Fettsäureester wie künstliches Entenbürzeldrüsenfett, Dibutylphthalat, Adipinsäurediisopropylester, letzterem verwandte Estergemische u.a.

Triglyceride wie Capryl/Caprinsäuretriglycerid, Triglyceridgemische mit Pflanzenfettsäuren der Kettenlänge $C_8$-$C_{12}$ oder anderen speziell ausgewählten natürlichen Fettsäuren, Partialglyceridgemische gesättigter und ungesättigter evtl. auch hydroxylgruppenhaltige Fettsäuren, Monodiglyceride der $C_8$/$C_{10}$-Fettsäuren und andere.

Fettalkohole wie Isotridecylalkohol, 2-Octyldodecanol, Cetylstearyl-alkohol, Oleylalkohol.

Fettsäuren wie Ölsäure.

Besonders gut geeignete spreitende Öle sind die folgenden:

Isopropylmyristat, Isopropylpalmitat, Capryl/Caprinsäureester von gesättigten Fettalkoholen der Kettenlänge $C_{12}$-$C_{18}$, wachsartige Fettsäureester wie künstliches Entenbürzeldrüsenfett.

D. Pflaster

Weitere topisch anwendbare Zubereitungen der vorliegenden Erfindung sind medizinische Pflaster zur Abgabe der Wirkstoffe der Formel I an die Haut über einen längeren Zeitraum.

Gegenstand der Erfindung sind demnach auch medizinische Pflaster zur Verabreichung eines Wirkstoffs der Formel I an die Haut, enthaltend eine Deckschicht, die aus einem längs-querelastischen, mit einem Polymer imprägnierten oder beschichteten, bevorzugt textilen Flächengebilde, insbesondere Gewirk oder Gestrick besteht, eine Reservoirschicht und eine abziehbare Schutzschicht, wobei die Reservoirschicht ein Polymer bestehend aus Polyisobutylen und/oder dessen Copolymerisate, ein Schleppmittel und ein Harz enthält.

Unter Polymer im Sinne dieses Teils der Erfindung werden bevorzugt Polyisobutylen und/oder dessen Copolymerisate verstanden.

Als Polyisobutylene im Sinne der Erfindung werden Polyisobutylene verstanden, die herstellungsgbedingt eine Molmassen-Verteilung $M_w/M_n$ von 1,5 bis 3,5, vorzugsweise 2,0 bis 3,0 und ein Viskositätsmittel der Molmasse - wiederum herstellungsbedingt - von 30.000 bis zu 4.000.000 g/Mol aufweisen. Vorzugsweise beträgt das Viskositätsmittel der erfindungsgemäß einzusetzenden Polyisobutylene 50.000 bis 1.000.000 g/Mol, besonders bevorzugt 80.000 bis 500.000 g/Mol. Die Viskositätsmittel lassen sich in bekannter Weise bestimmen gemäß Polymer-Handbook, J. Brandrup und F.H. Immergut, Wiley & Sons, New York, 1975, Kap. IV, S. 35.

Diese Polyisobutylene sind seit langem bekannt und können z.B. gemäß US-PS 2 203 873 oder gemäß DE-PS 704 038 mit sauren Katalysatoren hergestellt werden.

Copolymerisate des Isobutylens im Sinne der Erfindung sind die des Isobutylens mit 0,5 bis 5 Mol-% konjugierten Diolefinen, vorzugsweise solchen mit 4 bis 6 C-Atomen, wie z.B. Butadien-1,3, Piperylen, 2,3-Dimethylbutadien, besonders bevorzugt mit Isopren, deren Molmassen von 30.000 bis 200.000 g/Mol betragen können. Auch diese Isobuten-Copolymere sind bekannt. Ganz besondere bevorzugt werden Polyisobutylen-Homopolymerisate mit einem Viskositätsmittel von 80.000 bis 500.000 eingesetzt.

Unter Schleppmittel im Sinne der Erfindung werden Öle, Fettsäureester, Triglyceride, Alkohole und/oder Fettsäuren verstanden.

Unter Ölen, im Sinne des die medizinischen Pflaster betreffenden Teils der Erfindung, werden hochsiedende, aliphatische, araliphatische und/oder aromatische Kohlenwasserstoffe verstanden, vorzugsweise Paraffinöl, Purcellinöl, Perhydrosqualen und Lösungen von mikrokristallinen Wachsen in den Ölen, Mineralöle, bevorzugt Öle, deren Siedebereich zwischen 150°C und 400°C liegt; ferner ungesättigte Kohlenwasserstoffe mit mindestens 16 C-Atomen wie z.B. Oligomere von Monoolefinen wie Tetraisobutylen, Pentaisobutylen, Hexaisobutylen oder auch flüssige Polymerisate aus Dien(Monoen)-(Co)-Polymerisaten. Beispiele für flüssige Polymerisate aus konjugierten Dienen sind solche aus Butadien, Isopren, 1,3-Pentadien, 2,3-Dimethylbutadien, Copolymerisate verschiedener Diene sowie auch flüssige Copolymerisate aus einem konjugierten Diolefin und geringen Mengen von Monoolefinen wie z.B. Buten-1, Isobuten, Hexen-1, Octen-1, Styrol mit MG von 400 bis 6.000, vorzugsweise 800 bis 3.000 sowie Iodzahlen von 200 bis 500 und Viskositäten von 100 bis 10.000 cP bei 50°C.

Besonders bevorzugt sind flüssige Polybutadien-Polymerisate, die zu mindestens 90 % 1,4-verknüpft sind, deren Anteil an cis-Doppelbindungen mehr als 60 % beträgt und deren Molmassen 1.000 bis 4.000 betragen.

Unter Ölen werden auch Silikonöle verschiedener Viskosität, vorzugsweise mit mittleren Molgewichten von 312 bis 15.000, besonders bevorzugt Polydimethylsiloxane, verstanden.

Unter Fettsäureestern werden solche verstanden, die mindestens 12 C-Atome, vorzugsweise 15 bis 46 C-Atome, besonders bevorzugt 16 bis 36 C-Atome, enthalten.

Insbesondere werden darunter verstanden: Ethylstearat, Laurinsäurehexylester, Dipropylen-glykolpelargonat, Palmitinsäurecetylester, Isopropylmyristat, Isopropylpalmitat, Capryl/Caprinsäureester von gesättigten Fettalkoholen der Kettenlänge $C_{12}$-$C_{18}$, Isopropylstearat, Ölsäureoleylester, Ölsäuredecylester, Ethyloleat, künstliches Entenbürzeldrüsenfett, und zwar jeweils einzeln oder im Gemisch.

Unter Triglyceriden werden reine oder gemischte Ester des Glycerins mit Fettsäuren der Kettenlänge $C_8$-$C_{18}$ verstanden, vorzugsweise Capryl-und/oder Caprinsäuretriglyceride.

Unter Fettsäuren werden gesättigte oder ungesättigte Fettsäuren, vorzugsweise solche mit 12 bis 24 C-Atomen, einzeln oder im Gemisch miteinander, besonders bevorzugt Ölsäure, verstanden.

Unter Ölen im Sinne der Erfindung werden ferner verstanden:

Süßmandelöl, Avocadoöl, Sesamöl, Rizinusöl, Olivenöl, Traubenkernöl, Nelkenöl, Erdnußöl, Maisöl, Haselnußöl, Jojobaöl, Carthamaöl und Weizenkeimöl, jeweils einzeln oder im Gemisch.

Unter Harzen, im Sinne des die Pflaster betreffenden Teils der Erfindung, werden Kolophonium, dehydriertes Kolophonium, Glycerinester von dehydriertem Kolophonium, Glycerinester von Kolophoniumgummi, hydriertes Kolophonium, Glycerinester von hydriertem Kolophonium, Pentaerythritester von hydriertem Kolophonium, Methylester von hydriertem Kolophonium, polymerisiertes Kolophonium, Glycerinester von polymerisiertem Kolophonium, Terpenharze, Cumaron/Inden-Harze, hydrierte Petroleumharze, mit Maleinsäureanhydrid modifiziertes Kolophonium und Kolophoniumderivate, $C_5$-Petroleumharze und Halbester von Styrol/Maleinsäure-Co-polymeren einzeln oder im Gemisch miteinander verstanden. Besonders bevorzugt werden Polyterpenharze aus Alpha-bzw. Beta-Pinen oder modifizierte Glycerinester der Kolophoniums. Diese Harze können je nach den erforderlichen Eigenschaften hinsichtlich der Klebrigkeit und der Haftfestigkeit auf dem Teil, an dem das resultierende Pflaster angebracht werden soll, entweder allein oder in Kombination miteinander verwendet werden.

Die Wirkstoffe der Formel 2 können in den Pflastern der Erfindung in einer Menge von 1 bis 30 Gew.-%, vorzugsweise 2 bis 20 Gew.-% in die Reservoirschicht eingearbeitet werden. Die angegebenen Gew.-% beziehen sich auf Gesamtreservoir.

Den Wirkstoffen der Formel I können zusätzlich noch Wirksubstanzen zugesetzt werden oder auch kühlende oder duftabgebende Substanzen, vorzugsweise Methylsalicylat, Glycolsalicylat, Salicylsäure, Menthol, Pfefferminzöl, Kampfer, Thymol, Acrinol, Scopoliaextrakt, Chlorpheniraminmaleat, Benzylnicotinat, Capsicumextrakt, Nonylvanillylamin, Capsaicin.

Erforderlichenfalls können die erfindungsgemäßen Pflaster mit Additiven und Füllstoffen, z.B. Alterungsschutzmitteln, Antioxidantien und Verstärkungsfüllstoffen versetzt werden.

Als Deckschicht für die erfindungsgemäßen Pflaster wurden längs-und querelastische Gewirke und Gestricke eingesetzt (s. z.B. Koch-Satlow, Großes Textillexikon, Deutsche Verlagsanstalt Stuttgart 1965).

Gewirke und Gestricke sind demnach textile Flächengebilde, die aus einem oder mehreren Fadensystemen durch Maschenbildung auf Wirk-oder Strickmaschinen hergestellt werden. Man unterscheidet zwei Kategorien: Kuliergewirke und -gestricke (Hauptkennzeichen: Fadenverlauf in Querrichtung, analog Schußrichtung bei Geweben) sowie Kettengewirke (Hauptkennzeichen: Fadenverlauf in Längsrichtung, analog Kettrichtung bei Geweben).

Die in der Fachterminologie übliche Begriffstrennung in Gewirke und Gestricke bezieht sich auf den Herstellvorgang. Beim Wirken werden die Maschen einer Maschenreihe gleichzeitig ausgebildet (abgeschlagen), während beim Stricken eine Masche nach der anderen entsteht. Bei der Begriffszuordnung gibt es jedoch Ausnahmen. Bindungstechnisch unterscheiden sich Kulier-Gewirke und -gestricke nicht.

Gewirke und Gestricke besitzen im Gegensatz zu Geweben hohe Dehnung und Elastizität, besonders in Breitenrichtung; des weiteren haben sie infolge der Maschenstruktur ein großes Porenvolumen, was Luftdurchlässigkeit und Thermoisolation begünstigt. Diese und andere Eigenschaften können durch Bildung wie auch Faserstoff-und Garnauswahl weitgehend variiert werden.

Bevorzugt weisen die erfindungsgemäß verwendeten Gewirke und Gestricke Stretchcharakter auf. Zur Erzielung dieses Stretchcharakters werden die in der Textiltechnik üblichen Methoden eingesetzt (s. Koch-Satlow, Seite 441) oder aber es werden gleich bei der Auswahl der Grundmaterialien für die Gewirke und Gestricke Elastomerfasern bzw. Elastomergarne verwendet.

Neben den Gewirken und Gestricken lassen sich als Deckschicht für die erfindungsgemäßen Pflaster allgemein textile Flächengebilde mit Stretchcharakter verwenden, d.h. alle dreidimensionalen Gebilde aus natürlichen und synthetischen Textilfasern wie Geflechte, Vliesstoffe oder Filze sind als Deckschicht geeignet.

Als Basismaterial für die Deckschicht kommen u.a. Fasern und Filamente aus Polyamid, Polyester, Polyurethan, Polyamid-Polyurethan, Baumwolle, Zellwolle und tierische Wolle zur Anwendung.

Die textile Deckschicht der erfindungsgemäßen Pflaster wird imprägniert oder beschichtet. Zum Beschichten und Imprägnieren werden die üblichen Techniken und Materialien benutzt (s. auch Koch-Satlow, S. 157 bis 159 sowie S. 616 ff.).

Bevorzugt wird die Deckschicht mit Polyisobutylen imprägniert oder beschichtet. Dabei ist die Molmasse des Polyisobutylens bevorzugt >1.000.000 g/Mol (Viskositätsmittel).

Als Beschichtungs-und Imprägnierungsmaterial kommen bevorzugt die Polyisobutylene infrage, die auch in der Reservoirschicht enthalten sind, jedoch höhere Molekulargewichte und keine Klebrigkeit aufweisen.

Die Abziehfolie der erfindungsgemäße Pflaster kann aus okklusiven, flexiblen oder nichtflexiblen Materialien bestehen, wie Polyethylen, Polypropylen, Polyethylenterephthalat, Nylon u.ä. bekannte Folien. Als Abziehfolie können auch Metallfolien, wie Aluminiumfolie, allein oder mit Polymeren laminiert, angewandt werden. Auch mehrschichtige Folien wie Laminate aus Polyethylen mit Polyester-PE-terephthalat und mit Aluminium bedampft, können eingesetzt werden. Andere abziehbare Folien sind u.a. mit Silikon behandelte Polyester, Polyethylenterephthalat mit endständigen Silikongruppen, behandeltes Papier, mit Silikon behandeltes Papier, mit Polyethylen beschichtetes Papier u.ä.

## E. Gele

Die Erfindung betrifft bevorzugt auch topisch anwendbare Zubereitungen in Form von Gelen. Unter Gel werden dabei disperse Systeme "fest/flüssig" verstanden, bei denen die disperse Phase nicht mehr frei beweglich ist.

In Gelform vorliegende topische Zubereitungen der Wirkstoffe der Formel I eignen sich zur Behandlung von bakteriellen Infektionen von bevorzugt Körperhöhlen, insbesondere der Mundhöhle. Die Depot-Wirkung, gute Hafteigenschaften und eine höhere Bioverfügbarkeit der Wirkstoffe ermöglichen eine Kurzzeittherapie.

Um zu einer Verkürzung der Therapiedauer zu kommen, benötigt man eine gewisse Depot-Wirkung und eine höhere Bioverfügbarkeit der Wirkstoffe. Dazu sind besonders die erfindungsgemäßen Formulierungen in Gelform geeignet. Wenn man ohne weitere Erhöhung der Wirkstoffkonzentration eine Verkürzung der Therapiedauer erreichen will, muß man für eine optimale Bioverfügbarkeit des Wirkstoffes Sorge tragen.

Insbesondere im Bereich der Mundschleimhaut sind daher Formulierungen erforderlich, die einerseits nach Auftragen auf die Mundschleimhaut eine ausreichende Haftfähigkeit aufweisen, andererseits den in der Formulierung enthaltenden Wirkstoff ausreichend, auch in Speichel gelöst, freisetzen können.

Es wurde gefunden, daß solche Formulierungen der Wirkstoffe der Formel I in Kombination mit Kortikoidsteroiden, die als Gelbildner einen Celluloseether, insbesondere Hydroxypropylcellulose, Natriumalginat oder Propylenglykolalginat und außerdem die üblichen Formulierungshilfsstoffe enthalten, optimale Hafteigenschaften und eine optimale Freisetzung des Wirkstoffes und damit eine verkürzte Therapiedauer durch das Erreichen von antibakteriellen Konzentrationen des Wirkstoffes ermöglichen. Dieser Effekt wird dadurch erreicht, daß die Bioverfügbarkeit der in den Formulierungen enthaltenden Wirkstoffe, durch adhärierende Eigenschaften erhöht werden und dadurch die Wirkstoff-Freisetzung in Speichel gesteigert werden kann.

Wirkstoffe, die in dieser Weise formuliert werden können, sind alle Wirkstoffe der Formel I, insbesondere Ciprofloxacin und die übrigen auf der Seite 4 genannten Wirkstoffe. Sie sind in den erfindungsgemäßen Gelen bevorzugt in Mengen von 0,05 bis 30 Gew.-%, vorzugsweise 0,05 bis 0,5 Gew.-%, insbesondere 0,1 bis 1 Gew.-%, vorhanden. Die Kortikoidsteroidmengen entsprechen den oben angegebenen Mengen.

Als Gelbildner kommen solche makromolekularen Verbindungen infrage, die sich sowohl in Wasser als auch in organischen Lösungsmitteln lösen bzw. anquellen können. Vor allem seien hier Celluloseether genannt, von denen 2,5 bis 17,5 % benötigt werden.

Folgt man einer Einteilung der makromolekularen Hilfsstoffe, (Keipert et al., Die Pharmazie 28, 145-183 (1973)), so kommen vor allem ionische Makromoleküle in deren Salzform zur Anwendung. Dies sind unter anderem Natriumcarboxymethylcellulose, Polyacrylsäure, Polymethacrylsäure und deren Salze, Natriumamylopektinsemiglykolat, Alginsäure und Propylenglykol-Alginat als Natriumsalz, arabisches Gummi, Guar-Gummi.

Amphotere Makromoleküle wie Protein-Derivate, z.B. Gelatine sind ebenso geeignet wie nichtionische Polymere, z.B. Methylcellulose, Hydroxypropylcellulose und lösliche Stärken, die obige Anforderungen erfüllen.

Als Gelbildner, die auch stabilisierend wirken, kommen langkettige lineare hochmolekulare Polysaccharide mit einem Molekulargewicht von mehr als einer Million in Betracht. Von solchen Stabilisatoren werden 0,1 bis 1,5 % benötigt.

Als Lösungsmittel sind Wasser und auch alle mit Wasser mischbaren Lösungsmittel geeignet. In Betracht kommen z.B. Alkanole wie Ethanol und Isopropylalkohol, Benzylalkohol, Propylenglykol, u.a.

Als besonders stabile Gel-Kombinationsgrundlagen wurden solche makromolekularen Verbindungen gefunden wie z.B. Hydroxypropylcellulose (Molekulargewicht wahrscheinlich 2.000.000).

Bevorzugt enthalten die erfindungsgemäßen Gele 2,5 bis 35,0 Gew.-% der unter C. genannten Spreitmittel.

F. Puder

Erfindungsgemäße topisch anwendbare Zubereitungen in Puderform enthalten die Wirkstoffe der Formel I und Kortikosteroide und bevorzugt Pudergrundlagen wie Reis-, Mais-, Weizenstärke, Talcum, Bolus alba und Bolus rubra, Kieselgur, Aerosil, Magnesiumoxid, Magnesiumcarbonat, Zinkoxid und Titandioxid.

Algemein können die folgenden Puderrohstoffe erfindungsgemäß eingesetzt werden: Aerosil, Aluminium-hydroxid, -palmitat und -stearat, amylum non mucilaginosum, Arrowroot, Avicel microcrystalline Cellulose, Bariumsulfat, Bolus, Cab-o-sil, Calamine, Calcium carbonicum praecipitatum, Calcium-silikat, -stearat und -sulfat, Calflo, Dextrane, Ekasil, Flosilite, Glaxie Micron, Gasil, Kaolin, Kieselgur, Kieselsäure D 17, Kirusol, Kronos-Titandioxid-Pigmente, Laminarin, Luvokoll, Magnesiumcarbonat, -oxid, -silikat, -stearat und -trisilikat, Magnesol, Marinco, Metallseifen, Metasap, Milchzucker, NAL, Neosyl, Oracid-Pruder, Ottalume, Polyethylen, Polyamidpulver, Polyesterpulver, Pontybond 2150, Powdertrol, Quso, Reisstärke, Santocel C, Sea sorb, Seidenfibroin, Sicol, Siflox, Silicagel, Silin S 100, S-Micron Silica, Syloid, Talcum cetylatum, Talkum, Tego-Metallseifen, Tiotal, Tioxide, Titandioxid, Titanstearat, Veegum, Vinylon, Witcarb, Zink-carbonat, -myristat, -oxid und -stearat.

Stärke, insbesondere Reisstärke und Weizenstärke werden bevorzugt als Pudergrundlage für die Wirkstoffe der Formel I und der Kortikosteroide verwendet. Ebenso kommen veretherte Stärken bevorzugt zur Anwendung.

Die Herstellung von wirkstoffhaltigen Pudern erfolgt bei festen Wirkstoffen der Formel I und den Kortikosteroiden durch Mischen der Wirkstoffe in feinstverteilter Form mit der entsprechenden Menge an Pudergrundlage nach den Gesichtspunkten des Mischens von Pulvern.

Sie lassen sich auch zusammen mit verflüssigten Treibgasen in Aerosol-Druckgaspackungen abfüllen und als sprühbare Puder verwenden. Hierzu sind etwa 8 bis 9 Gewichtsanteile Treibmittel auf 1 Teil Puder zu verwenden.

Flüssige oder halbfeste Wirkstoffe der Formel I und die Kortikosteroide werden mit einem kleinen Anteil der Grundlage innig verrieben und die dann trockene Masse mit dem Rest der Pudergrundlage gemischt. Eventuell lassen sich auch flüchtige Lösungsmittel als Hilfsstoffe einsetzen, um die Wirkstoffe gleichmäßig auf die Pudergrundlage aufzuziehen.

G. Suspensionen

Erfindungsgemäße Suspensionen enthalten die Wikstoffe der Formel I sowie der Kortikosteroide in fester Form dispergiert in einer flüssigen Phase. Die Teilchengröße der dispergierten Wirkstoffe liegt in den Grenzen zwischen 0,1 µm bis etwa 100 µm. Je nach Gebrauch liegt der Feststoffanteil einer erfindungsgemäßen Suspension etwa zwischen 0,5 % und 40 %. Ihre grundlegende Eigenschaft sollte die langsame Sedimentation der Feststoffpartikel und deren leichte Aufschüttelbarkeit sein, um Fehldosierungen zu vermeiden.

Erfindungsgemäße Suspensionen werden in der Regel durch Anreiben des Feststoffes mit einer kleinen Menge Flüssigkeit und allmähliches Verdünnen bis zum Endvolumen hergestellt. Besteht das Dispersionsmittel aus mehreren Flüssigkeiten (z.B. Mucilago Tylose, Glycerin und Wasser), so wird die Komponente mit der höchsten Viskosität zum Anreiben verwendet. Die endgültige Mischung kann maschinell mit Hilfe von Mixern, Homogenisatoren, Korundscheibenmühlen, Perlmühlen, Ultraschall u.a. homogenisiert werden.

Die folgenden Maßnahmen dienen zur Herstellung stabiler erfindungsgemäßer Suspensionen.

Erhöhung der Benetzungsfähigkeit der Partikeln und gezielte Flockung durch Zugabe von amphiphilen Hilfsstoffen und geeigneten Elektrolyten.

Erhöhung der Viskosität des Dispersionsmittels zur Verringerung der Sedimentationsgeschwindigkeit.

Zur Erniedrigung der Grenzflächenspannung fest/flüssig lassen sich wie bei den Emulsionen Tenside einsetzen.

Diese werden von den hydrophoben Partikeln der dispersen Phase so adsorbiert, daß ihre solvatisierten hydrophilen Molekülteile vom Wirkstoff weg in das Dispersionsmittel hineinragen. Werden ionogene Tenside eingesetzt, so erhalten die Teilchen neben einer guten Benetzung auch eine ausreichende elektrostatische Aufladung, die die Stabilisierung der Suspension unterstützt.

Elektrolyte wie Phosphate, Alkalicarbonate, Citrate, Gallate u.a., die nach Adsorption an die Wirkstoffteilchen potentialbestimmend sind, führen zu einem hohen Zeta-Potential und bewirken so die gegenseitige Abstoßung.

Mittels der Wirkstoffe der Formel I und der Kortikosteroide lassen sich leicht wieder aufschüttelbare und damit gleichmäßig zu dosierende Suspensionen dadurch herstellen, daß man die durch ein anionogenes Tensid zunächst negativ geladenen ursprünglich hydrophoben Partikeln mittels eines Calcium-oder Aluminiumsalzes zur Flockung bringt. Das sich bildende lockere, sperrige Sediment kann durch Schütteln leicht redispergiert werden.

Der Zusatz von makromolekularen Stoffen wie Methylcellulose, Stärke, Tragant u.a. bewirkt neben der Viskositätserhöhung den Aufbau von Sorbathüllen um die Partikeln der dispersen Phase. Dies wiederum erschwert die Aggregation.

Besonders wirksam sind Gelbildner, die der äußeren Phase thixotrope Eigenschaften verleihen. Das in Ruhelage ausgebildete Gelgerüst verhindert die Sedimentation der dispersen Phase. Unter der Einwirkung geringer Scherkräfte (Umschütteln) wird das Dispersionsmittel frei fließend, und die Suspension kann gut dosiert werden.

Das für wäßrige Suspensionen Gesagte gilt vice versa auch für ölige Suspensionen. Hier sind vor allem bei Verarbeitung oxophiler (hydrophiler) Feststoffe Tenside z.B. vom Typ der Metallseifen angezeigt. Häufig aber genügt bereits der Anteil von freien Fettsäuren im Öl oder ein entsprechender Zusatz zur Lyophilisierung von Zinkoxid oder anderen arzneilich verwendeten hydrophilen Stoffen. Die Wirkung eines so netzenden Zusatzes ist bereits am Aussehen z.B. eines Zinköls zu erkennen: Während nicht benetztes Zinkoxid aufstockend sedimentiert und einen glänzenden Überstand von reinem Öl ergibt (v.a. bei Einsatz von flüssigem Paraffin), werden die mit Hilfe von Fettsäuren benetzten ZnO-Partikeln Flocken bilden, wenig sedimentieren und der Oberfläche ein mattes Aussehen verleihen.

H. Emulsionen

Die erfindungsgemäßen topischen Zubereitungen der Wirkstoffe der Formel I und der Kortikosteroide können auch in Form von "flüssig/ flüssig" dispersen Systemen vorliegen.

Nach "Arzneiformenlehre", P.H. List, Wissenschaftliche Verlagsgesellschaft mbH, Stuttgart 1985, Seite 168 sind für die Herstellung von Emulsionen folgende Punkte zu berücksichtigen:

1. Geforderter Emulsionstyp - O/W, W/O, bikohärentes System
2. Viskosität - fließfähiges oder streichbares System
3. Temperatur-Stabilität - Stabilität in möglichst großem Temperaturbereich, Sterilisierbarkeit
4. Zerteilungsgrad - Tröpfchengröße >1 µm für gewöhnliche Emulsionen

5. Bestandteile - Auswahl der Komponenten aus therapeutischen, physiologischen, technologischen und wirtschaftlichen Gründen

6. Konzentrationsverhältnisse-aus anwendungsspezifischen Gründen

7. Geräte und Ansatzgrößen

Aufgrund der Vielfalt der Faktoren ist es nicht möglich, allgemein gültige Herstellungsregeln aufzustellen. Es sind vielmehr Versuchsreihen durchzuführen, wobei das HLB-System, das Verfahren von Lin zur Bereitung von O/W-Emulsionen, die Erstellung von Dreikomponentendiagrammen oder die Bildung von Emulgatorgelen hilfreich sein können.

Emulgatoren und andere Hilfsmittel zur Herstellung von erfindungsgemäßen topischen Zubereitungen wurden in H.P. Fiedler, Lexikon der Hilfsstoffe für Pharmazie, Kosmetik und angrenzende Gebiete, Editio Cantor K.G., Aulendorf i. Württ., 1971, insbesondere auf den Seiten 185 - 194 beschrieben.

Emulsionen der Wirktoffe der Formel I in Kombination mit den Kortikosteroiden können der äußerlichen Anwendung dienen. Äußerlich sind beide Emulsionstypen, d.h. O/W-und W/O-Emulsionen anwendbar.

Flüssige O/W-Emulsionen, die oft auch als Lotionen bezeichnet werden, dienen vorwiegend dermatologischen Zwecken. Die halbfesten Formen werden als abwaschbare Salben bezeichnet; ebenso die halbfesten W/O-Emulsionen, die man Cremes nennt. Flüssige W/O-Emulsionen zur äußerlichen Anwendung führte DAB 6 noch unter der Bezeichnung Linimente.

I. Lösungen

Zu den erfindungsgemäßen topisch anwendbaren Zubereitungen der Wirkstoffe der Formel I und der Kortikosteroide gehören Lösungen.

Die Auswahl eines Lösungsmittel oder eines Lösungsmittelgemisches für die Herstellung der erfindungsgemäßen Zubereitungen hängt primär von der Natur des zu lösenden Stoffes oder Stoffgemisches ab. Darüber hinaus aber spielt die Frage eine Rolle, ob das Lösungmittel ganz oder teilweise in der fertigen Zubereitung verbleibt. Im Falle des Verbleibens muß das Lösungsmittel in erster Linie physiologisch unbedenklich sein.

Aus diesen Gründen kommt für die Arzneibereitung nur eine relativ geringe Anzahl von Flüssigkeiten als Lösungsmittel in Frage.

Die bei weitem am häufigsten gebrauchte ist Wasser.

Als Lösungsmittel sind daher Wasser und auch alle mit Wasser mischbare Lösungsmittel geeignet. In Betracht kommen z.B. Alkohole wie Ethanol und Isopropylalkohol, Propylenglykol, Polyethylenglykole, Glycerin, Methylcellosolve, Cellosolve, Ester, Morpholine, Dioxan, Dimethylsulfoxid etc.

Es können bei der Herstellung der erfindungsgemäßen Formulierungen ein oder mehrere Lösungsmittel eingesetzt werden. Als Lösungsvermittler eignen sich vor allem:

Tenside wie polyoxyethylierte Sorbitanfettsäureester, polyoxyethylierte Fettsäure-ether und -ester, etc. Daneben kommen auch nicht wassermischbare Lösungsmittel in Frage wie flüssige Ester und Öle, insbesondere Isopropylmyristat, Isopropylpalmitat, 2-Octyldodecanol, mittelkettige Triglyceride, Adipinsäureester, Sebacinsäureester, Paraffinöl und Silikonöl sowie deren Mischungen, aber auch mit Wasser mischbare Lösungsmittel und Tenside.

J. Salben, Pasten, Cremes, Schäume

(Nach Ullmann, Bd. 18, Pharmazeutische Technologie und Arzneiformenlehre, P.H. List, 4. Auflage, Wissenschaftl. Verlagsgesellschaft mbH, Stuttgart)

Salben sind Gele von plastischer Verformbarkeit, die Wirkstoffe der Formel I und Kortikosteroide gelöst, emulgiert oder suspendiert enthalten können.

Sie dienen der lokalen Behandlung erkrankter Haut-oder Schleimhautpartien. Auch hier können sie die Funktion von Schutz-und Decksalben erfüllen. Enthalten die Salben Wirkstoffe der Formel I und Kortikosteroide, die in die tiefergelegenen Hautschichten eindringen, penetrieren, und dort zur Wirkung gelangen, so spricht man von Penetrationssalben. Als Resorptionssalben werden solche bezeichnet, deren Wirkstoffe nach Penetration in das Unterhautgewebe schließlich in den Blutkreislauf gelangen (percutane oder transcutane Resorption).

Entsprechend ihrer Zusammensetzung, ihrer Konsistenz oder nach dem Applikationsort werden Salben verschieden benannt:

Unguenta, Salben (engl.: ointments; franz.: pommades) ist entweder der übergeordnete Begriff für diese

Arzneiform oder meint wasserfreie Zubereitungen auf der Basis von verschiedenen Grundlagen.

Cerata ist die Bezeichnung für Salben, die aus einer Mischung von Wachs und Öl bestehen.

Cremores, Cremes sind Salben besonders weicher Konsistenz, die größere Mengen an Wasser enthalten.

Glycerola werden glycerolhaltige Zubereitungen von halbfester Konsistenz bezeichnet.

Pastae, Pasten sind Salben mit einem hohen Anteil puderförmiger Feststoffe und deshalb hoher Konsistenz.

Oculenta, Unguenta ophthalmica, Augensalben (engl.: eye ointments) sind weiche Salben zur Applikation in den Bindehautsack und an den Lidrändern, an die bezüglich Reinheit und Teilchengröße besondere Anforderungen gestellt werden.

Salbengrundlagen, die für die topisch anwendbaren Zubereitungen der Erfindung zur Anwendung kommen, sind Kohlenwasserstoffgele, Lipogele, Hydrogele, Polyethylenglykolgele und Silikongele.

Bevorzugt kommen Vaselin, Plastibase, Wachse (nach DGF), insbesondere Bienenwachs, Walrat DAB 8. Cetaceum, Woll wachs DAB 8. Lanae Cera, Ölsäureoleylester DAB 8. Oleyli oleas, Isopropylmyristat, Schweineschmalz, Gehärtetes Erdnußöl, Glycerol, Sorbitlösungen, niedrigmolekulare Polyethylenglykole, kolloide Kieselsäure, Aerosil, Quelltone wie Bentonite, Kaliseife, Schmierseife, Opodeldoc, Stärke, Cellulosederivate, Polyacrylsäure, Polyethylenglykolgele DAB 8, Siliconöle in Betracht.

Die erfindungsgemäßen topischen Zubereitungen kommen in Salbenform als Lösungssalben, Emulsionssalben und Suspensionssalben zur Anwendung. Sie enthalten vorzugsweise Antioxidantien wie Avenol, Avenex, Conidendrin, Nor-Conidendrin, Nordihydroguajaritsäure, Tocopherol, Ascorbinsäureester z.B. -stearat, -palmitat, -myristat, -laurat, 3-Butyl-4-hydroxyanisol, Hydrochinon, Propylgallat, Citronensäure, cis-Methylmaleinsäure, Gallussäureester wie Ethylgallat, Propylgallat, Jonol, BHT, Tetraoxydimethylbisphenyl (TDBP).

Pasten sind hochkonzentrierte Suspensionen mit Fließgrenze zur Anwendung auf der Haut oder Schleimhaut. Sie enthalten einen großen Anteil unlöslicher Pulver, die in einem flüssigen oder salbenartigen Vehikel dispergiert sind.

Die pulvrigen Bestandteile mit einer Korngröße von maximal 100 µm werden allmählich mit dem nötigenfalls auf dem Wasserbad geschmolzenen oder erweichten Dispersionsmittel zu einer gleichmäßigen Masse angerieben und möglichst mit einer Salbenmühle homogenisiert.

Cremes im Sinne der Erfindung sind besonders geschmeidige Zubereitungen, die größere Wassermengen in Form von Öl-in-Wasser-oder Wasser-in-Öl-Emulsionen enthalten. Das Verhältnis von wäßriger zu öliger Phase bestimmt die Viskosität und die Verstreichbarkeit einer Creme. Geeignete Grundlagen je nach Emulsionstyp sind z.B. verschiedene Lanette-Typen (Natriumfettalkoholsulfat), Cetylstearylalkohol oder Wollfett, Wollwachsalkohole usw.

Die erfindungsgemäßen topischen Zubereitungen können auch in Form von Schäumen vorliegen. Unter Schäumen werden dabei disperse Systeme "gasförmig/flüssig" verstanden (Definitionen, Begriffe und Herstellung siehe "Arzneiformenlehre", List).

Die erfindungsgemäßen topischen Zubereitungen zeigen bei geringer Toxizität ein breites antibakterielles Spektrum gegen gram-positive und gram-negative Keime, insbesondere gegen Enterobakteriaceen sowie antiinflammatorische Eigenschaften; vor allem auch gegen solche, die resistent sind gegen verschiedene Antibiotika, wie z.B. Penicilline, Cephalosporine, Aminoglykoside, Sulfonamide, Tetracycline.

Die erfindungsgemäßen Zubereitungen sind gegen ein sehr breites Spektrum von Mikroorganismen wirksam. Mit ihrer Hilfe können gram-negative und gram-positive Bakterien und bakterienähnliche Mikroorganismen bekämpft sowie die durch diese Erreger hervorgerufenen Erkrankungen verhindert, gebessert und/oder geheilt werden.

Cremes im Sinne der Erfindung sind besonders geschmeidige Zubereitungen, die größere Wassermengen in Form von Öl-in-Wasser-oder Wasser-in-Öl-Emulsionen enthalten. Das Verhältnis von wäßriger zu öliger Phase bestimmt die Viskosität und die Verstreichbarkeit einer Creme. Geeignete Grundlagen je nach Emulsionstyp sind z.B. verschiedene Lanette-Typen (Natriumfettalkoholsulfat), Cetylstearylalkohol oder Wollfett, Wollwachsalkohole usw.

Die erfindungsgemäßen topischen Zubereitungen können auch in Form von Schäumen vorliegen. Unter Schäumen werden dabei disperse Systeme "gasförmig/flüssig" verstanden (Definitionen, Begriffe und Herstellung siehe "Arzneiformenlehre", List).

Die erfindungsgemäßen topischen Zubereitungen zeigen bei geringer Toxizität ein breites antibakterielles Spektrum gegen gram-positive und gram-negative Keime, insbesondere gegen Enterobakteriaceen sowie antiinflammatorische Eigenschaften; vor allem auch gegen solche, die resistent sind gegen verschiedene Antibiotika, wie z.B. Penicilline, Cephalosporine, Aminoglykoside, Sulfonamide, Tetracycline.

Die erfindungsgemäßen Zubereitungen sind gegen ein sehr breites Spektrum von Mikroorganismen

wirksam. Mit ihrer Hilfe können gram-negative und gram-positive Bakterien und bakterienähnliche Mikroorganismen bekämpft sowie die durch diese Erreger hervorgerufenen Erkrankungen verhindert, gebessert und/oder geheilt werden.

Besonders wirksam sind die erfindungsgemäßen topischen Zubereitungen gegen Bakterien und bakterienähnliche Mikroorganismen und empfindliche Prozesse sowie andere Kortikosteroid-Indikationen. Sie sind daher besonders gut zur Prophylaxe und Chemotherapie von lokalen und gegebenenfalls systemischen Infektionen in der Human-und Tiermedizin geeignet, die durch diese Erreger hervorgerufen werden.

Beispielsweise können lokale und/oder systemische Erkrankungen behandelt und/oder verhindert werden, die durch die folgenden Erreger oder durch Mischungen der folgenden Erreger verursacht werden: Gram-positive Kokken, z.B. Staphylokokken (Staph. aureus, Staph. epidermidis) und Streptokokken (Strept. agalactiae, Strept. faecalis, Strept. pneumoniae, Strept. pyogenes); gram-negative Kokken (Neisseria gonorrhoeae) sowie gram-negative Stäbchen wie Enterobakteriaceen, z.B. Escherichia coli, Hämophilus influenzae, Citrobacter (Citrob. freundii, Citrob. divernis), Salmonella und Shigella; ferner Klebsiellen (Klebs. pneumoniae, Klebs. oxytoca), Enterobacter (Ent. aerogenes, Ent. agglomerans), Hafnia, Serratia (Serr. marcescens), Proteus (Pr. mirabilis, Pr. rettgeri, Pr. vulgaris), Providencia, Yersinia, sowie die Gattung Acinetobacter. Darüber hinaus umfaßt das antibakterielle Spektrum die Gattung Pseudomonas (Ps. aeruginosa, Ps. maltophilia) sowie strikt anaerobe Bakterien wie z.B. Bacteroides fragilis, Vertreter der Gattung Peptococcus, Peptostreptococcus sowie die Gattung Clostridium; ferner Mykoplasmen (M. pneumoniae, M. hominis, M. urealyticum) sowie Mykobakterien, z.B. Mycobacterium tuberculosis.

Die obige Aufzählung von Erregern ist lediglich beispielhaft und keineswegs beschränkend aufzufassen. Als Krankheiten, die durch die genannten Erreger oder Mischinfektionen verursacht und durch die erfindungsgemäßen topisch anwendbaren Zubereitungen verhindert, gebessert oder geheilt werden können, seien beispielsweise genannt:

Infektionskrankheiten beim Menschen wie zum Beispiel septische Infektionen, Knochen-und Gelenkinfektionen, Hautinfektionen, postoperative Wundinfektionen, Abszesse, Phlegmone, Wundinfektionen, infizierte Verbrennungen, Brandwunden, Infektionen im Mundbereich, Infektionen nach Zahnoperationen, septische Arthritis, Mastitis, Tonsillitis, Genital-Infektionen und Augeninfektionen.

Außer beim Menschen können bakterielle Infektionen auch bei anderen Spezies behandelt werden. Beispielhaft seien genannt:

Schwein: Coli-diarrhoe, Enterotoxämie, Sepsis, Dysenterie, Salmonellose, Metritis-Mastitis-Agalaktiae-Syndrom, Mastitis;

Wiederkäuer (Rind, Schaf, Ziege): Diarrhoe, Sepsis, Bronchopneumonie, Salmonellose, Pasteurellose, Mykoplasmose, Genitalinfektionen;

Pferd: Bronchopneumonien, Fohlenlähme, puerperale und postpuerperale Infektionen, Salmonellose;

Hund und Katze; Bronchopneumonie, Diarrhoe, Dermatitis, Otitis, Harnwegsinfekte, Prostatitis;

Geflügel (Huhn, Pute, Wachtel, Taube, Ziervögel und andere): Mycoplasmose, E. coli-Infektionen, chronische Luftwegserkrankungen, Salmonellose, Pasteurellose, Psittakose.

Ebenso können bakterielle Erkrankungen bei der Aufzucht und Haltung von Nutz-und Zierfischen behandelt werden, wobei sich das antibakterielle Spektrum über die vorher genannten Erreger hinaus auf weitere Erreger wie zum Beispiel Pasteurella, Brucella, Campylobacter, Listeria, Erysipelothrix, Corynebakterien, Borellia, Treponema, Nocardia, Rikettsien, Yersina, erweitert.

Zur vorliegenden Erfindung gehören pharmazeutische Zubereitungen, die neben nicht-toxischen, inerten pharmazeutisch geeigneten Trägerstoffen eine oder mehrere erfindungsgemäße Verbindungen enthalten oder die aus einem oder mehreren erfindungsgemäßen Wirkstoffen bestehen, sowie Verfahren zur Herstellung dieser Zubereitungen.

Zur vorliegenden Erfindung gehören auch pharmazeutische Zubereitungen in Dosierungseinheiten.

Die oben aufgeführten pharmazeutischen Zubereitungen können außer den erfindungsgemäßen Verbindungen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

## Beispiele

### Haftende topische Zubereitungen

1. Beispiel für einen Stift

Zusammensetzung

| | | |
|---|---|---|
| 1) | Ciprofloxacinhydrochlorid x 1 $H_2O$ | 0,2 g |
| 2) | Wirkstoff x | 1,0 g |
| 3) | Bienenwachs | 12,0 g |
| 4) | Vaseline | 6,0 g |
| 5) | Cetylalkohol | 4,0 g |
| 6) | Wollwachs | 3,0 g |
| 7) | Isopropylmyristat | 9,0 g |
| 8) | Rizinusöl | 64,8 g |
| | | 100,0 g |

Herstellung

Die Substanzen 3) bis 8) werden eingewogen und bei ca. 70° unter Rühren geschmolzen. Darin wird Ciprofloxacinhydrochlorid suspendiert. Die Schmelze wird in entsprechenden Formen (Stifte) gegossen und auf Raumtemperatur abgekühlt.

2. Beispiel einer Schutzsalbe

Zusammensetzung

| | | |
|---|---|---|
| 1) | Ciprofloxacin | 0,5 g |
| 2) | Wirkstoff H | 0,5 g |
| 3) | niedermolekulares Acrylharz | 5,0 g |
| 4) | Wasser demin. | 71,5 g |
| 5) | Ammoniak | 1,0 g |
| 6) | Wollwachsalkohol | 9,0 g |
| 7) | Tween 81® | 1,0 g |
| 8) | Paraffin flüssig | 11,5 g |
| | | 100,0 g |

Herstellung

a) Die Substanzen 1, 2, 6, 7, 8 auf 65°C unter Rühren erwärmen.
b) Die Substanzen 3 und 4 (wäßrige Phase) auf 65°C erwärmen.
c) b in a unter starkem Rühren einarbeiten.
d) c unter starkem Rühren den Ammoniak zufügen und unter Rühren auf Raumtemperatur abkühlen.

3. Beispiel eines filmbildenden Konzentrates

Zusammensetzung

| | |
|---|---|
| 1) Ciprofloxacin | 5,0 g |
| 2) Wirkstoff G[1] | 1,0 g |
| 3) Natriumalginat | 9,0 g |
| 4) nichtionischer Emulgator | 2,0 g |
| 5) kolloidale Kieselsäure | 2,0 g |
| 6) Isopropanol ad 100 ml | 81,0 g |
| | 100,0 g |

Herstellung

Ciprofloxacin und Hydrocortisonacetat werden unter Rühren in 3 und 5 dispergiert. In die Dispersion werden 2 und 4 eingetragen und homogenisiert.

Vor Gebrauch wird diese Suspension aufgeschüttelt und mit Wasser 1 + 9 verdünnt. Es bildet sich ein Gel, das nach dem Trocknen einen elastischen Film auf der Haut bildet, der längere Zeit auch bei Wasserkontakt haftet.

4. Beispiel einer Lösung

Zusammensetzung

| | |
|---|---|
| 1) Ciprofloxacin oder Norfloxacin + 0,5 g Wirkstoff H | 0,03 g |
| 2) Poly(methylvinylether/maleinsäure-monoalkylester) | 2,5 g |
| 3) Isopropanol | 96,97 g |
| | 100,0 g |

Herstellung

Die Lösung kann auf die Haut aufgetragen werden. Nach dem Trocknen bildet sich ein wirkstoffhaltiger Film auf der Haut.

5. Beispiel für eine Spray-Formulierung

Die Lösung aus Beispiel 4 wird mit Propan/Butan als Treibgas im Verhältnis 1 + 2 in geeignete Aerosoldosen abgefüllt.

Pflaster

Beispiel 1

Eine 12,5 %ige Polyisobutylenlösung (M. G. Viskositätsmittel 1.270.000) (in Benzin), wird auf silikonisiertem Papier aufgetragen und einem Gewirk bestehend aus Polyamid-Polyurethanfasern zukaschiert und im Trockenkanal stufenweise bei 70 / 90 / 100°C getrocknet (Polymer 30 g/m²).

Auf silikonisiertem Papier wird eine in Benzin/Aceton gelöste Mischung, bestehend aus
36,000 g Polyisobutylen M.G. Viskositätsmittel 400.000,
44,928 g Paraffin dünnflüssig,
9,000 g Polyterpenharz aus β-Pinen,
10,000 g Ciprofloxacin oder Ofloxacin oder Norfloxacin
0,072 g Alterungsschutzmittel
aufgetragen und im Trockenkanal stufenweise bei 70 / 90 / 100°C getrocknet (Wirkstoffabgabesystem ca. 150 g/m²).

Nach dem Trocknen wird das mit Polyisobutylen imprägnierte Gewirk mit Stretchcharakter zukaschiert.

Beispiel 2

Auf silikonisiertem Papier wurde eine Polymerlösung (Benzin/Aceton), bestehend aus
36,000 g Polyisobutylen M.G. Viskositätsmittel 1.270.000,
44,928 g Paraffin dünnflüssig,
9,000 g Polyterpenharz aus α-Pinen,
10,000 g Ciprofloxacin und 5,000 g Wirkstoff N
0,072 g Alterungsschutzmittel
aufgetragen und im Trockenkanal stufenweise bei 70 / 90 / 110°C getrocknet.

Nach dem Trocknen wurde das Wirkstoffabgabesystem mit Polyisobutylen (wie in Beispiel 1) auf beschichtetes Stretchmaterial kaschiert.

Die erfindungsgemäßen Pflaster besitzen eine ebenso gute Resorption der Wirkstoffe wie herkömmliche mit Aluminium-Polyethylen-Folien beschichtete Pflaster.

Zum Kaschieren des Wirkstoffdepots auf die textilen Flächengebilde mit Stretchcharakter werden u.a. die Methoden und Materialien eingesetzt, die in Koch-Satlow, Großes Textillexikon, beschrieben werden.

Gele

Beispiel 1
Ciprofloxacinlactat 0,20 g
Wirkstoff Prednisolon 5,00 g
Benzylalkohol 3,00 g
Hydroxypropylcellulose (M.G. 1.000.000) 2,50 g
Wasser entmineralisiert ad 100 g

Beispiel 2
Ciprofloxacinlactat 0,20 g
Hydrocortisonacetat 0,25 g
Benzylalkohol 3,00 g
Hydroxypropylcellulose (M.G. 1.000.000) 2,5 g
Wasser entmineralisiert ad 100 g

Beispiel 3
Norfloxacinhydrochlorid 0,20 g
Prednisolon 5,00 g
Benzylalkohol 3,00 g
Hydroxypropylcellulose (M.G. 1.000.000) 2,5 g
Wasser entmineralisiert ad 100 g

Beispiel 4 Ofloxacinhydrochlorid 0,20 g
Prednisolon 5,00 g
Benzylalkohol 3,00 g
Hydroxypropylcellulose (M.G. 1.000.000) 17,50 g
Wasser entmineralisiert ad 100 g

Cremes

Beispiel 1 Creme,O/W

Phase I Sorbitanmonostearat 2,0 g
Polyoxyethylen (20)-sorbitanmonostearat 1,5 g
Walrat künstlich 3,0 g
Cetylstearylalkohol 10,0 g
2-Octyldodecanol 13,5 g
Auf 75°C erwärmen, rühren, mischen.

Phase II Ciprofloxacin, Norfloxacin oder Ofloxacin, Enoxacin, Pefloxacin 1,0 g
in Verbindung mit 1 bis 2 g der Wirkstoffe A bis Z und $A^1$ bis $H^1$
zur Phase I geben, rühren, suspendieren.

Phase III Benzylalkohol 1,0 g
Wasser, entmineralisiert 68,0 g
auf 75°C erwärmen und zur Phase II geben. Intensiv mischen, langsam unter weiterem Rühren auf Raumtemperatur abkühlen. Homogenisieren.

Beispiel 2 Creme,O/W

Phase I Sorbitanmonostearat 1 - 3 g
Polyoxyethylen (20)-sorbitanmonostearat 0,5 - 2,5 g
Walrat künstlich 2 - 4 g
Cetylstearylalkohol 5 - 15 g
Isopropylmyristat 5 - 25 g
Auf 75°C erwärmen, rühren, mischen.

Phase II Ciprofloxacin, Norfloxacin oder Ofloxacin 0,5 - 1,5 g
und Wirkstoffe A bis Z bzw. $A^1$ bis $H^1$ in eben solchen Mengen zur Phase I geben, rühren, suspendieren.

Phase III Benzylalkohol 0,5 - 1,5 g
Wasser, entmineralisiert quant. sat.
auf 75°C erwärmen und zur Phase II geben. Intensiv mischen, langsam unter weiterem Rühren auf Raumtemperatur abkühlen. Homogenisieren.

Flüssig-Pflaster

Beispiel 1 Flupameson 1,0 g
Ciprofloxacin 1,0 g
Benzylalkohol 5,0 g
Hydroxypropylcellulose (M.G. 60.000) 10,0 g
Isopropanol ad 100 ml

Beispiel 2Triamcinolon 0,1 g
Ciprofloxacin 0,1 g
Benzylalkohol 5,0 g
Isopropylmyristat 6,0 g
Hydroxypropylcellulose (M.G. 60.000) 10,0 g
Isopropanol ad 100 ml

Beispiel 3Fluocinolonacetonid 1,0 g
Ciprofloxacin 1,0 g
Benzylalkohol 4,0 g
Isopropylstearat 10,0 g
Hydroxypropylcellulose (M.G. 60.000) 12,0 g
Isopropanol ad 100 ml

Beispiel 4Betamethasonvalerat 1,0 g
Ciprofloxacin 1,0 g
1,2-Propylenglykol 1,0 g
Isopropylmyristat 6,0 g
Hydroxypropylcellulose (M.G. 60.000) 10,0 g
Isopropanol ad 100 ml

Beispiel 5Norfloxacin 0,1 g
Benzylalkohol 5,0 g
Isopropylmyristat 6,0 g
Hydroxypropylcellulose (M.G. 60.000) 10,0 g
Isopropanol ad 100 ml

Beispiel 6Ofloxacin 1,0 g
Benzylalkohol 5,0 g
Isopropylmyristat 6,0 g
Hydroxypropylcellulose (M.G. 60.000) 10,0 g
Isopropanol ad 100 ml

Beispiel 7Prednisolon 1,0 g
Ciprofloxacin 1,0 g
Benzylalkohol 8,0 g
Isopropylmyristat/Isopropylstearat/Isopropylpalmitat 1,0 g
Hydroxypropylcellulose (M.G. 60.000) 10,0 g
Isopropanol ad 100 ml

Beispiel 8Prednisolon 1,0 g
Norfloxacin 1,0 g
Benzylalkohol 5,0 g
Isopropylmyristat 6,0 g
Methylcellulose 10,0 g
Isopropanol ad 100 ml

Sprays

Die nach den Beispielen 1 bis 8 hergestellten Wirkstofflösungen bzw. Suspensionen können auch zu Sprays verarbeitet werden. Zu diesem Zweck vermischt man z.B. 60 bis 90 % Wirkstofflösung mit 20 bis 40 % der gebräuchlichen Treibmittel, z.B. $N_2$, $N_2O$, $CO_2$, Propan, Butan, Halogenkohlenwasserstoff usw.

Emulsionen/Cremes

Beispiel 1 Emulsion, O/W.

Phase I "Glycerylstearate" (Gemisch von Mono-und Diglyceriden der Palmitin-und Stearinsäure) 8,00 g
2-Octyldodecanol 10,00 g
Cetylstearylalkohol mit ca. 12 Mol Ethylenoxid 1,50 g
Cetylstearylalkohol mit ca. 30 Mol Ethylenoxid 1,50 g
Paraffin, dickflüssig 6,00 g
1,2-Propylenglykol 5,00 g
Capryl/Caprinsäure-triglycerid 6,00 g
Die Mischung wird gerührt und bei 70°C geschmolzen.

Phase II Wasser, entmineralisiert 58,00 g

Phase III Prednisolon 1,00 g
Ciprofloxacin 1,00 g
suspendiert in Benzylalkohol 3,00 g

Zu auf 75°C erwärmten Phase II wird die auf 70°C erwärmte Phase I unter Rühren hinzugefügt. Man läßt langsam auf 40°C abkühlen, gibt Phase III zu und läßt unter Rühren auf Raumtemperatur abkühlen. Die Rohemulsion wird bei 20 bis 25°C im Hochdruckhomogenisator homogenisiert. In analoger Weise werden die Beispiele 2 und 3 verarbeitet.

Beispiel 2 Emulsion, O/W. Desoxycorticosteron 5,00 g
Ciprofloxacin 1,00 g
"Glycerylstearate" Gemisch von Mono-und Diglyceriden der Palmitin-und Stearinsäure 8,00 g
Diglyceriden der Palmitin-und Stearinsäure 9,00 g
Cetylstearylalkohol mit ca. 12 Mol Ethylenoxid 3,00 g
2-Octyldodecanol 10,00 g
Paraffin, dickflüssig 5,00 g
Benzylalkohol 5,00 g
Wasser entmineralisiert ad 100 ml

Beispiel 3 Emulsion, O/W. Desoxycorticosteron 5,00 g
Norfloxacin 1,00 g
"Glycerylstearate" Gemisch von Mono-und Diglyceriden der Palmitin-und Stearinsäure 9,00 g
Cetylstearylalkohol mit ca. 12 Mol Ethylenoxid 3,00 g
2-Octyldodecanol 10,00 g
Benzylalkohol 5,00 g
Isopropylmyristat 5,00 g
Wasser entmineralisiert ad 100 ml

Beispiel 4 Creme, weiche Konsistenz O/W Prednisolon, Fluocinolonacetonid, Hydrocortisonacetat 1,00 g
Ciprofloxacin, Norfloxacin, Ofloxacin 1,00 g
"Glycerylstearate" Gemisch von Mono-und Diglyceriden der Palmitin-und Stearinsäure 4,00 g
Cetylpalmitat 4,00 g
Cetylstearylalkohol mit ca. 12 Mol Ethylenoxid 1,00 g
Cetylstearylalkohol mit ca. 30 Mol Ethylenoxid 1,00 g
Isopropylmyristat/Isopropylpalmitat/Isopropylstearat-Gemisch 5,00 g
Schwach vernetzte Polyacrylsäure von extrem hohem M.G. 0,50 g
Natriumhydroxid 45 %ig 0,11 g
Glycerin 3,00 g
Benzylalkohol 3,00 g
Wasser entmineralisiert ad 100 ml

Beispiel 5 Creme, weiche Konsistenz O/W Wirkstoffe A bis Z bzw. $A^1$ bis $H^1$ jeweils 1,00 g
Ciprofloxacin, Norfloxacin, Ofloxacin 1,00 g
"Glycerylstearate" Gemisch von Mono-und Diglyceriden der Palmitin-und Stearinsäure 4,00 g
Cetylpalmitat 4,00 g
Cetylstearylalkohol mit ca. 12 Mol Ethylenoxid 1,00 g
Cetylstearylalkohol mit ca. 30 Mol Ethylenoxid 1,00 g
Isopropylmyristat/Isopropylpalmitat, Isopropylstearat-Gemisch 5,00 g
Schwach vernetzte Polyacrylsäure von extrem hohem M.G. 0,50 g
Natriumhydroxid 45 %ig 0,11 g
Glycerin 3,00 g
Benzylalkohol 3,00 g
Wasser entmineralisiert ad 100 ml

Beispiel 6 Creme, weiche Konsistenz O/W Hydrocortisonacetat 1,00 g
Norfloxacin, Ofloxacin 1,00 g
"Glycerylstearate" Gemisch von Mono-und Diglyceriden der Palmitin-und Stearinsäure 4,00 g
Stearinsäure 4,00 g
Cetylpalmitat 4,00 g
Cetylstearylalkohol mit ca. 12 Mol Ethylenoxid 1,00 g
Cetylstearylalkohol mit ca. 30 Mol Ethylenoxid 1,00 g
Isopropylmyristat/Isopropylpalmitat, Isopropylstearat-Gemisch 5,00 g
Schwach vernetzte Polyacrylsäure von extrem hohem M.G. 0,50 g
Natriumhydroxid 45 %ig 0,11 g
Glycerin 3,00 g
Benzylalkohol 3,00 g
Wasser entmineralisiert ad 100 ml

Beispiel 7 Emulsion, nichtfettend O/W

Phase I Ölsäuredecylester 2,50 g
Isopropylmyristat 2,50 g
Paraffin, dünnflüssig 4,00 g
Polyethylenstearat 0,90 g
Sorbitan-und Glycerinfettsäureester 0,60 g
Die Mischung wird 10 Minuten bei 70°C gerührt und geschmolzen.

Phase II Wasser, entmineralisiert 50,00 g
Allantoin 0,10 g

Carbopolschleim Alkohol, vergällt 10,00 g
Carbopol 934 (schwach vernetzte Polyacrylsäure) 0,70 g
Wasser, entmineralisiert 22,945 g

Man dispergiert mit Turrax, läßt 2 Stunden quellen und neutralisiert dann mit 0,155 g 45 %iger Natronlauge.

Zur 75°C heißen Phase II gibt man die 70°C warme Phase I unter Rühren hinzu und kühlt auf 45°C ab. Bei 45°C Carbopol-Schleim einrühren und bis 40°C weiter abkühlen. Bei 40°C 1,00 g Collagen zugeben und auf 25°C abkühlen. 1,0 g Ciprofloxacin in 3,0 g Benzylalkohol einarbeiten und zur Phase I und II zugeben.

Anschließend wird die Rohemulsion unter Rühren bei 20°C bis 25°C im Hochdruckhomogenisator homogenisiert.

In analoger Weise werden die Beispiele 8, 9, 10, 11 verarbeitet.

Beispiel 8 Emulsion, nichtfettend O/W Hydrocortisonacetat 0,20 g
Ciprofloxacin 0,10 g
Ölsäuredecylester 2,50 g
Isopropylmyristat 2,50 g
Paraffin, dünnflüssig 4,00 g
Polyethylenstearat 0,90 g
Sorbitan-und Glycerinfettsäureester 0,60 g
Allantoin 0,155 g
Schwach vernetzte Polyacrylsäure von extrem hohem Molekulargewicht 0,70 g
Collagen 1,00 g
Benzylalkohol 3,00 g
Ethanol 10,00 g
Wasser, entmineralisiert ad 100 ml

Beispiel 9 Emulsion, nichtfettend O/W Hydrocortisonacetat 0,15 g
Norfloxacin 0,15 g
Ölsäuredecylester 2,50 g
Isopropylmyristat 2,50 g
Paraffin, dünnflüssig 4,00 g
Polyethylenstearat 0,90 g
Sorbitan-und Glycerinfettsäureester 0,60 g
Allantoin 0,10 g
Schwach vernetzte Polyacrylsäure von extrem hohem Molekulargewicht 0,70 g
Natriumhydroxid 45 %ig 0,155 g
Collagen 1,00 g
Benzylalkohol 3,00 g
Ethanol 10,00 g
Wasser, entmineralisiert ad 100 ml

Beispiel 10 Emulsion, nichtfettend O/W Hydrocortisonacetat 0,10 g
Ofloxacin 0,10 g
Ölsäuredecylester 2,50 g
Isopropylmyristat 2,50 g
Paraffin, dünnflüssig 4,00 g
Polyethylenstearat 0,90 g
Sorbitan-und Glycerinfettsäureester 0,60 g
Allantoin 0,10 g
Natriumhydroxid 45 %ig 0,155 g
Schwach vernetzte Polyacrylsäure von extrem hohem Molekulargewicht 0,70 g
Collagen 1,00 g
Benzylalkohol 3,00 g

Ethanol 10,00 g
Parfümöl 0,60 g
Wasser, entmineralisiert ad 100 ml

Beispiel 11 Emulsion, nichtfettend O/W Hydrocortisonacetat, Prednisolon jeweils 0,05 g
Ciprofloxacin 0,05 g
Ölsäuredecylester 2,50 g
Isopropylmyristat 2,50 g
Paraffin, dünnflüssig 4,00 g
Polyethylenstearat 0,90 g
Sorbitan-und Glycerinfettsäureester 0,60 g
Allantoin 0,10 g
Natriumhydroxid 45 %ig 0,155 g
Schwach vernetzte Polyacrylsäure von extrem hohem Molekulargewicht 0,70 g
Collagen 1,00 g
Benzylalkohol 3,00 g
Ethanol 10,00 g
Wasser, entmineralisiert ad 100 ml

Beispiel 12 Emulsion, nichtfettend O/W Triamcinolon 0,50 g
Ciprofloxacin 0,10 g
"Glycerylstearate" Gemisch von Mono-und Diglyceriden der Palmitin-und Stearinsäure 4,00 g
Cetylpalmitat 4,00 g
Cetylstearylalkohol mit ca. 12 Mol Ethylenoxid 1,00 g
Cetylstearylalkohol mit ca. 30 Mol Ethylenoxid 1,00 g
Isopropylmyristat/Isopropylpalmitat, Isopropylstearat-Gemisch 5,00 g
Schwach vernetzte Polyacrylsäure von extrem hohem Molekulargewicht 0,50 g
Natriumhydroxid 45 %ig 0,11 g
Glycerin 3,00 g
Benzylalkohol 3,00 g
Wasser entmineralisiert ad 100 ml

Beispiel 13 Creme, weiche Konsistenz O/W Flupameson 0,05 g
Ofloxacin 0,05 g
"Glycerylstearate" Gemisch von Mono-und Diglyceriden der Palmitin-und Stearinsäure 4,00 g
Cetylpalmitat 4,00 g
Cetylstearylalkohol mit ca. 12 Mol Ethylenoxid 1,00 g
Cetylstearylalkohol mit ca. 30 Mol Ethylenoxid 1,00 g
Isopropylmyristat/Isopropylpalmitat, Isopropylstearat-Gemisch 5,00 g
Schwach vernetzte Polyacrylsäure von extrem hohem Molekulargewicht 0,50 g
Natriumhydroxid 45 %ig 0,11 g
Glycerin 3,00 g
Benzylalkohol 3,00 g
Wasser entmineralisiert ad 100 ml

Beispiel 14 Creme, weiche Konsistenz O/W Prednisolon 5,00 g
Norfloxacin, Pefloxacin 1,00 g
"Glycerylstearate" Gemisch von Mono-und Diglyceriden der Palmitin-und Stearinsäure 4,00 g
Na-Stearat 16,00 g
Cetylstearylalkohol mit ca. 12 Mol Ethylenoxid 3,00 g
Benzylalkohol 3,50 g
2-Octyldodecanol 2,50 g
Kokosfettsäure-Isopropylester 2,50 g

(Isopropylmyristat/Isopropylpalmitat, Isopropylstearat-Gemisch)
Paraffin, dünnflüssig 3,00 g
Wasser entmineralisiert ad 100 ml

Gele

Beispiel 1

Phase I Hydrocortisonacetat 1,00 g
Ofloxacin 1,00 g
in Isopropanol 40,00 g
lösen; anschließend
Polyol-Fettsäureester 4,00 g
Benzylalkohol 3,00 g
Adipinsäurediisopropylester 4,00 g
einrühren und lösen.

Phase II in entmineralisiertes Wasser 46,10 g
Carbopol 940 1,50 g
unter Rühren einbringen, ca. 2 h quellen lassen und mit
NaOH 45 %ig 0,40 g
neutralisieren,
Phase I, portionsweise, langsam unter Rühren in Phase II einarbeiten.

In analoger Weise wird bei den Beispielen 2 und 3 gearbeitet.

Beispiel 2Flupameson 1,00 g
Ciprofloxacin 1,00 g
Polyol-Fettsäureester 4,00 g
Isopropylmyristat 4,00 g
Benzylalkohol 1,00 g
Isopropanol 45,00 g
Carbopol 940 1,50 g
NaOH 45 %ig 0,40 g
Wasser, entmineralisiert 43,10 g

Beispiel 3Fluocinolonacetonid 1,00 g
Ofloxacin 1,00 g
Polyol-Fettsäureester 4,00 g
Kokosfettsäure-Isopropylester 4,00 g
Benzylalkohol 5,00 g
Isopropanol 45,00 g
Carbopol 940 1,50 g
NaOH 45 %ig 0,40 g
Wasser, entmineralisiert 39,10 g

Fettsalbe

Beispiel 1Wirkstoffe A bis Z bzw. $A^1$ bis $H^1$ 1,00 g
Ciprofloxacin 1,00 g
Wollwachsalkohole 5,00 g
Vaseline weiß ad 100 g

Puder

Beispiel 1Wirkstoffe A bis Z bzw. $A^1$ bis $H^1$ 0,1 g
Ciprofloxacinhydrochlorid 0,1 g
Lactose ad 100 g

SchüttelmixturWirkstoffe A bis Z bzw. $A^1$ bis $H^1$ 1,0 g
Ciprofloxacin 1,0 g
Lanette N 3,0 g
Zinkoxid 18,0 g
Talkum 18,0 g
Glycerin 85 % 18,0 g
Ethanol 96 % 13,2 g
Wasser entmineralisiert 28,8 g
Ciprofloxacin, Zinkoxid und Talkum werden in einer Lösung der übrigen Hilfsstoffe suspendiert.

PasteWirkstoffe A bis Z bzw. $A^1$ bis $H^1$ 10,0 g
Ciprofloxacin 10,0 g
Weizenstärke 20,0 g
Zinkoxid 20,0 g
Weißes Vaselin 50,0 g

Die Feststoffe werden 4 Stunden lang bei 40°C getrocknet, gesiebt, in der geschmolzenen Vaseline suspendiert und kaltgerührt.

PolyethylenglykolsalbeWirkstoffe A bis Z bzw. $A^1$ bis $H^1$ 0,50 g
Ciprofloxacin 0,50 g
Polyethylenglykol 300 49,75 g
Polyethylenglykol 1500 49,75 g

Ciprofloxacin wird in der Schmelze der Polyethylenglykole suspendiert. Danach wird kaltgerührt.

FettsalbeWirkstoffe A bis Z bzw. $A^1$ bis $H^1$ 1,00 g
Ciprofloxacin mikrofein 1,00 g
p-Hydroxybenzoesäuremethylester 0,07 g
p-Hydroxybenzoesäuremethylester 0,03 g
Paraffin dickflüssig 15,00 g
Wollwachsalkoholsalbe 83,90 g

Ciprofloxacin wird in die geschmolzene Fettphase eingetragen. Danach wird unter Rühren abgekühlt.

Puder Wirkstoffe A bis Z bzw. A[1] bis H[1] 0,01 g
Ciprofloxacin, Natriumsalz 0,01 g
Reisstärke nicht quellbar 99,99 g

W/O-Salbe Wirkstoffe A bis Z bzw. A[1] bis H[1] 2,00 g
Ciprofloxacin 2,00 g
Protegin X 22,00 g
Bienenwachs 3,00 g
Isopropylmyristat 1,50 g
Mittelkettige Triglyceride 1,50 g
Gemisch alkylverzweigter Fettsäureester 2,00 g
Glycerin 3,00 g
Wasser entmineralisiert 65,00 g

Weitere Beispiele für insbesonders penetrierende Zubereitungen

Beispiel 1 Triamcinolonacetonid 1,0 %
(Triamcinolon in folgenden)
Ciprofloxacinhydrochlorid 1,0 %
Propylene Glycol (1,2-propanediol) 94,0 %
Methyllaurat 4,0 %

Beispiel 2 Hydrocortisonacetat 1,0 %
Ciprofloxacinlactat 1,0 %
Propylene Glycol (1,2-propanediol) 93,0 %
Ölsäure 5,0 %

Beispiel 3 Betamethasonvalerate 0,5 %
Ciprofloxacin 0,5 %
Propylene Glycol (1,2-propanediol) 93,0 %
Oleyl alcohol 6,0 %

Beispiel 4 Fluocinolonacetonid 0,5 %
Ciprofloxacin 0,5 %
Propylene Glycol (1,2-propanediol) 94,0 %
Monoolein 5,0 %

Beispiel 5 Flupamesone 0,5 %
Ciprofloxacin 0,5 %
Propylene Glycol (1,2-propanediol) 97,0 %
Myristylalkohol 2,0 %

Beispiel 6 Triamcinolon 0,5 %
Ofloxacin 0,5 %
1,2-Butanediol 95,0 %
Methyllaurat 4,0 %

Beispiel 7Triamcinolon 0,5 %
Norfloxacin 0,5 %
1,3-Butanediol 97,0 %
Methyllaurat 2,0 %

Beispiel 8Hydrocortisonacetat 0,25 %
Ciprofloxacin 0,25 %
1,2-Butanediol 97,50 %
Ölsäure 2,0 %

Beispiel 9Hydrocortisonacetat 2,0 %
Norfloxacin 2,0 %
1,3-Butanediol 91,0 %
Ölsäure 5,0 %

Beispiel 10Betamethasonvalerat 2,0 %
Pefloxacin, Ofloxacin 2,0 %
1,2-Butanediol 91,0 %
Oleylalkohol 5,0 %

Beispiel 11Fluocinolonacetonid 5,0 %
Ciprofloxacin 5,0 %
1,2-Butanediol 87,0 %
Monoolein 3,0 %

Beispiel 12Flupameson 1,0 %
Ciprofloxacin 1,0 %
Hydrocortisonacetat 1,0 %
Propylenglycol (1,2-Propanediol) 92,0 %
Myristylalkohol 5,0 %

Beispiel 13Desoxycorticosteron 5,0 %
Ciprofloxacin, Ofloxacin 5,0 %
Propylenglycol (1,2-Propanediol) 85,0 %
Ölsäure 5,0 %

Beispiel 14Prednisolon 5,0 %
Ciprofloxacin 5,0 %
Propylenglycol (1,2-Propanediol) 86,0 %
Myristylalkohol 4,0 %

Beispiel 15Prednison 2,0 %
Ciprofloxacin 2,0 %
1,2-Butanediol 52,0 %
Ölsäure 4,0 %
Ethanol 40,0 %

Beispiel 16Methylprednisolon 4,0 %
Ciprofloxacin 4,0 %
1,3-Butanediol 51,0 %
Oleylalkohol 1,0 %
Isopropanol 40,0 %

## Ansprüche

Topisch anwendbare Zubereitungen, enthaltend 0,05 bis 30 Gew.-% antibakteriell wirksamer Verbindungen der allgemeinen Formel

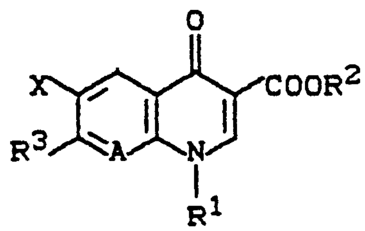

(I)

in welcher

$R^1$ für Methyl, Ethyl, Propyl, Isopropyl, Cyclopropyl, Vinyl, 2-Hydroxyethyl, 2-Fluorethyl, Methoxy, Amino, Methylamino, Dimethylamino, Ethylamino, Phenyl, 4-Fluorphenyl, 2,4-Difluorphenyl,

$R^2$ für Wasserstoff, Alkyl mit 1 bis 4 Kohlenstoffatomen, (5-Methyl-2-oxo-1,3-dioxol-4-yl)-methyl,

$R^3$ für Methyl oder eine cyclische Aminogruppe wie

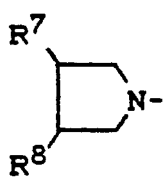 , 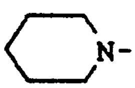 , 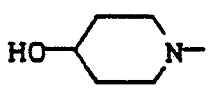 , 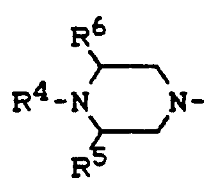 ,

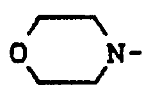 , 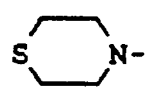 , 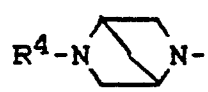 , 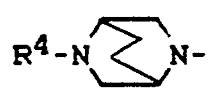 ,

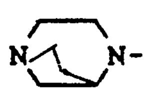 , 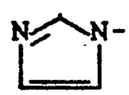 , 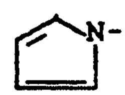

stehen, worin

$R^4$ für Wasserstoff, Alkyl mit 1 bis 4 Kohlenstoffatomen, 2-Hydroxyethyl, Allyl, Propargyl, 2-Oxopropyl, 3-Oxobutyl, Phenacyl, Formyl, $CFCl_2$-S-, $CFCl_2$-$SO_2$-, $CH_3O$-CO-S-, Benzyl, 4-Aminobenzyl,

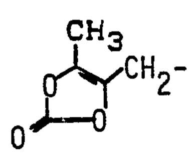

$R^5$ für Wasserstoff, Methyl,

$R^6$ für Wasserstoff, Alkyl mit 1 bis 4 Kohlenstoffatomen, Phenyl, Benzyloxymethyl,

$R^7$ für Wasserstoff, Amino, Methylamino, Ethylamino, Aminomethyl, Methylaminomethyl, Ethylaminomethyl, Dimethylaminomethyl, Hydroxy, Hydroxymethyl,

$R^8$ für Wasserstoff, Methyl, Ethyl oder Chlor stehen,

X für Wasserstoff, Fluor, Chlor oder Nitro und
A für N oder C-$R^9$ stehen, worin
$R^9$ für Wasserstoff, Halogen wie Fluor oder Chlor, Methyl oder Nitro steht,
oder
A auch gemeinsam mit $R^1$ eine Brücke der Struktur $-O-CH_2-CH-CH_3$, $-S-CH_2-CH-CH_3$ oder $-CH_2-CH_2-CH-CH_3$
bilden kann und
0,01 bis 10 Gew.-% eines Kortikosteroids.

2. Topisch anwendbare Zubereitungen, enthaltend 0,05 bis 30 Gew.-% Ciprofloxacin, Norfloxacin, Pefloxacin, Amifloxacin, Pirfloxacin, Ofloxacin und/oder Enoxacin und 0,01 bis 10 Gew.-% eines Kortikosteroids aus der Gruppe bestehend aus

A Beclomethasondipropionat
B Clobetasolpropionat
C Diflucortolonvalerat
D Fluocinolonacetonid
E Beclomethasondipropionat
F Betamethasonbenzoat
G Betamethasondipropionat
H Betamethasonvalerat
I Desonid
J Desoxymethason
K Diflorasondiacetat
L Diflucortolonvalerat
M Fluclorolonacetonid
N Fluocinolonacetonid
O Fluocinonid
P Fluocortolon
Q Flupredniden (Fluprednyliden)acetat
R Flurandrenolon
S Halcinonid
T Hydrocortisonbutyrat
X Triamcinolonacetonid
Y Clobetasonbutyrat
Z Flumethasonpivalat
$A^1$ Fluocinolonacetonid
$B^1$ Fluocortinbutylester
$C^1$ Fluocortolon
$D^1$ Flurandrenalon
$E^1$ Hydrocortison (Harnstoff)
$F^1$ Dexamethason
$G^1$ Hydrocortison (Alcohol oder Acetat)
$H^1$ Methylprednisolon

3. Topisch anwendbare Zubereitungen in Form von Lösungen, Sprays, Lotionen, Gelen, Salben, Cremes, Pulver, Pudersprays, Pasten, Suspensionen, Emulsionen, Schäumen, Stiften, Pflastern, Sprühpflastern, Okklusivverbänden, Umschlägen und gesteuerten Abgabesystemen, enthaltend 0,05 bis 30 Gew.-% eines oder mehrerer Wirkstoffe der Formel 1 gemäß Anspruch 1 und 0,01 bis 10 Gew.-% eines Kortikosteroids.

4. Topisch anwendbare Zubereitungen nach den Ansprüchen 1 bis 3, dadurch gekennzeichnet, daß sie
a) 0,1 - 20 %, vorzugsweise 0,1 - 5 %, eines Wirkstoffs der Formel I nach Anspruch 1,
a$^1$) 0,01 bis 10 Gew.-%, bevorzugt 0,02 bis 5 Gew.-% eines Kortikoidsteroids,
b) 1 - 40 %, vorzugsweise 1 - 20 %, eines wasserlöslichen Gel-oder Lack-bildenden Polymers,
c) 40 - 98 %, vorzugsweise 60 - 90 %, eines organischen, wassermischbaren Lösungsmittels, das schneller verdunstet als Wasser und in dem sich das Polymer nicht löst,
d) 0,1 - 10 Gew.-%, verschiedener Additiva, z.B. Weichmacher, Suspendierhilfsmittel, Antioxydantien, Spreitmittel, Farbstoffe etc.
enthalten.

5. Topisch anwendbare Zubereitungen nach den Ansprüchen 1 bis 4, enthaltend ein oder mehrere Spreitmittel.

6. Topisch anwendbare Zubereitungen in Form von Pflastern enthaltend eine Deckschicht, die aus einem längs-querelastischen, mit einem Polymer imprägnierten oder beschichteten, bevorzugt textilen Flächengebilde, insbesondere Gewirk oder Gestrick besteht, eine Reservoirschicht, die 0,05 bis 30 Gew.-% der Wirkstoffe der Formel I gemäß Anspruch 1 und 0,01 bis 10 Gew.-% eines Kortikosteroids enthält, und eine abziehbare Schutzschicht, wobei die Reservoirschicht ein Polymer bestehend aus Polyisobutylen und/oder dessen Copolymerisate, ein Schleppmittel und ein Harz enthält.

7. Topisch anwendbare Zubereitungen in Gelform, die 0,05 bis 30 Gew.-% der Wirkstoffe der Formel I gemäß Anspruch 1 und 0,01 bis 10 Gew.-% eines Kortikosteroids und als Gelbbildner Celluloseether, Polyacrylsäure, Polymethacrylsäure, Natriumalginat oder Propylenglykolalginat, Natriumamylopektinsemiglykolat, Alginsäure, arabisches Gummi, Guar-Gummi sowie gegebenenfalls lineare hochmolekulare Polysaccharide als stabilisierende Gelbbildner enthalten.

8. Topisch anwendbare Zubereitungen in Form von Suspensionen, enthaltend 0,05 bis 30 Gew.-% der Wirkstoffe der Formel I gemäß Anspruch 1 und 0,01 bis 10 Gew.-% eines Kortikosteroids in Teilchengrößen von 0,1 µm bis 100 µm und einen Feststoffanteil zwischen 0,5 bis 40 %.

9. Topisch anwendbare Zubereitungen in Form von Emulsionen, enthaltend 0,05 bis 30 Gew.-% der Wirkstoffe gemäß Anspruch 1 und 0,01 bis 10 Gew.-% eines Kortikosteroids.

10. Topisch anwendbare Zubereitungen in Form von Lösungen, enthaltend 0,05 bis 30 Gew.-% der Wirkstoffe gemäß Anspruch 1 und 0,01 bis 10 Gew.-% eines Kortikosteroids und ein Lösungsmittel aus der Gruppe bestehend aus Ethanol, Isopropylalkohol, Propylenglykol, Polyethylenglykole, Glycerin, Methylcellosolve, Cellosolve, Ester, Morpholine, Dioxan, Dimethylsulfoxid, Wasser oder Cyclohexanon sowie gegebenenfalls einen Lösungsvermittler.